# EUROPEAN PATENT APPLICATION

(11) **EP 4 596 530 A1**
(43) Date of publication of application: **06.08.2025**
(21) Application number: 23872592.3
(22) Date of filing: 29.09.2023
(51) Int. Cl.: C07C 231/12, C07C 235/78, C07D 311/66

(54) **OXIDATIVE CLEAVAGE METHOD OF TROLOX AMIDE**

(30) Priority: 30.09.2022 JP 2022158569
(71) Applicant: PTC Therapeutics, Inc., Warren, NJ 07059 (US)
(72) Inventor: USUTANI, Hirotsugu, Osaka-shi, Osaka 554-0022 (JP); YAMAMOTO, Kenji, Osaka-shi, Osaka 554-0022 (JP); HIRABAYASHI, Jun, Oita-shi, Oita 870-0106 (JP)
(74) Representative: J A Kemp LLP
(86) International application number: PCT/JP2023/035593
(87) International publication number: WO 2024/071372

(57) **Abstract**

Provided is a method of oxidatively cleaving Trolox amide, which is a method of manufacturing a compound of formula I: 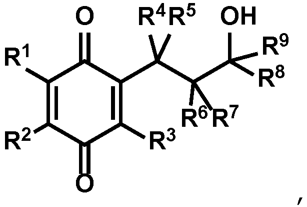 comprising the steps of:
contacting, in a solvent, an oxidizing agent with a compound of formula II: 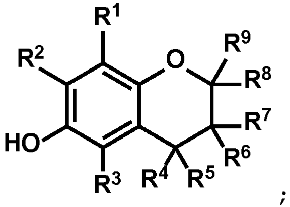 and
contacting a base with the solvent to promote a reaction between the compound of formula II and the oxidizing agent; wherein R¹, R², R³, R⁴, R⁵, R⁶, R⁷, R⁸, and R⁹ each independently represent a hydrogen atom, halogen, optionally substituted C₁₋₆ alkyl, optionally substituted C₁₋₆ alkoxy, or -C(=O)NR^{A}R^{B}, and R^{A} and R^{B} each independently represent a hydrogen atom, optionally substituted C₁₋₆ alkyl, or optionally substituted C₁₋₆ alkoxy.

## Description

### FIELD OF THE INVENTION

The present disclosure provides a method of oxidatively cleaving Trolox amide.

### BACKGROUND OF THE INVENTION

International Publication No. WO 2009/061744 describes that racemic 2-hydroxy-2-methyl-4-(2,4,5-trimethyl-3,6-dioxocyclohexa-1,4-dienyl)butanamide, which is useful in treating and/or suppressing a mitochondrial disorder and a specific pervasive developmental disorder, is synthesized from racemic Trolox (6-hydroxy-2,5,7,8-tetramethylchroman-2-carboxylic acid).

International Publication No. WO 2021/167095 describes an optically resolved Trolox intermediate and a method for producing same.

### Citation List

### Patent Literature

[PTL 1] International Publication No. WO 2009/061744
[PTL 2] International Publication No. WO 2021/167095

### SUMMARY OF THE INVENTION

### Solution to Problem

As a result of diligent studies, the inventors completed the present disclosure by discovering a novel method of oxidatively cleaving a Trolox amide related compound.

For example, the present disclosure provides the following items.

### (Item 1)

A method of manufacturing a compound of formula I: 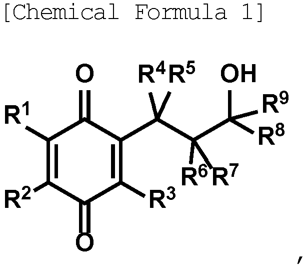 comprising the steps of:
contacting, in a solvent, an oxidizing agent with a compound of formula II: 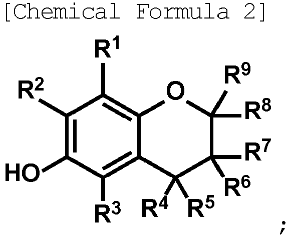 and
contacting a base with the solvent to promote a reaction between the compound of formula II and the oxidizing agent;
wherein
R¹, R², R³, R⁴, R⁵, R⁶, R⁷, R⁸, and R⁹ each independently represent a hydrogen atom, halogen, optionally substituted C₁₋₆ alkyl, optionally substituted C₁₋₆ alkoxy, or -C(=O)NR^{A}R^{B}, and
R^{A} and R^{B} each independently represent a hydrogen atom, optionally substituted C₁₋₆ alkyl, or optionally substituted C₁₋₆ alkoxy.

### (Item 1)

The method of item 1, wherein R¹ is a hydrogen atom or optionally substituted C₁₋₆ alkyl.

### (Item 3)

The method of item 1 or 2, wherein R¹ is methyl.

### (Item 4)

The method of any one of items 1 to 3, wherein R² is a hydrogen atom or optionally substituted C₁₋₆ alkyl.

### (Item 5)

The method of any one of items 1 to 4, wherein R² is methyl.

### (Item 6)

The method of any one of items 1 to 5, wherein R³ is a hydrogen atom or optionally substituted C₁₋₆ alkyl.

### (Item 7)

The method of any one of items 1 to 6, wherein R³ is methyl.

### (Item 8)

The method of any one of items 1 to 7, wherein R⁴ is a hydrogen atom or optionally substituted C₁₋₆ alkyl.

### (Item 9)

The method of any one of items 1 to 8, wherein R⁴ is a hydrogen atom.

### (Item 10)

The method of any one of items 1 to 9, wherein R⁵ is a hydrogen atom or optionally substituted C₁₋₆ alkyl.

### (Item 11)

The method of any one of items 1 to 10, wherein R⁵ is a hydrogen atom.

### (Item 12)

The method of any one of items 1 to 11, wherein R⁶ is a hydrogen atom or optionally substituted C₁₋₆ alkyl.

### (Item 13)

The method of any one of items 1 to 12, wherein R⁶ is a hydrogen atom.

### (Item 14)

The method of any one of items 1 to 13, wherein R⁷ is a hydrogen atom or optionally substituted C₁₋₆ alkyl.

### (Item 15)

The method of any one of items 1 to 14, wherein R⁷ is a hydrogen atom.

### (Item 16)

The method of any one of items 1 to 15, wherein R⁸ is a hydrogen atom, optionally substituted C₁₋₆ alkyl, or -C(=O)NR^{A}R^{B}.

### (Item 17)

The method of any one of items 1 to 16, wherein R⁸ is - C(=O)NR^{A}R^{B}.

### (Item 18)

The method of any one of items 1 to 17, wherein R⁹ is a hydrogen atom or optionally substituted C₁₋₆ alkyl.

### (Item 19)

The method of any one of items 1 to 18, wherein R⁹ is methyl.

### (Item 20)

The method of any one of items 1 to 19, wherein R^{A} and R^{B} each represent a hydrogen atom.

### (Item 21)

The method of any one of items 1 to 20, wherein the solvent is a polar aprotic solvent.

### (Item 22)

The method of any one of claims 1 to 20, wherein the solvent is a polar protic solvent.

### (Item 23)

The method of any one of items 1 to 20, wherein the solvent is selected from the group consisting of isopropyl acetate, ethyl acetate, tetrahydrofuran, 2-methyl-tetrahydrofuran, acetone, toluene, anisole, methyl tert-butyl ether, cyclopentyl methyl ether, methanol, ethanol, 2-propanol, 4-methyltetrahydropyran, acetonitrile, N-methylpyrrolidone, and dimethylsulfoxide.

### (Item 24)

The method of any one of items 1 to 23, wherein the solvent is dimethylsulfoxide.

### (Item 25)

The method of any one of items 1 to 24, wherein the oxidizing agent comprises a compound comprising iron.

### (Item 26)

The method of any one of items 1 to 25, wherein the oxidizing agent is iron(III) chloride or iron(III) nitrate.

### (Item 27)

The method of any one of items 1 to 26, wherein the oxidizing agent is iron(III) chloride hexahydrate.

### (Item 28)

The method of any one of items 1 to 27, wherein the base is selected from the group consisting of sodium hydroxide, potassium hydroxide, calcium hydroxide, barium hydroxide, potassium carbonate, sodium carbonate, sodium hydrogen carbonate, triethylamine, diisopropylethylamine, and ammonia water.

### (Item 29)

The method of any one of items 1 to 28, wherein the base is selected from the group consisting of sodium hydroxide, potassium carbonate, sodium carbonate, sodium hydrogen carbonate, triethylamine, and ammonia water.

### (Item 30)

The method of item 29, wherein the base is sodium hydroxide.

### (Item 31)

The method of any one of items 1 to 30, wherein the step of contacting the oxidizing agent is performed in a flow reactor.

### (Item 32)

The method of item 31, wherein an inner diameter of the flow reactor is about 0.1 mm to about 100 mm in the step of contacting the oxidizing agent.

### (Item 33)

The method of item 31 or 32, wherein a residence time of the step of contacting the oxidizing agent is 60 seconds or less.

### (Item 34)

The method of item 33, wherein the residence time is 0.1 seconds to 10 seconds.

### (Item 35)

The method of any one of items 1 to 34, wherein the step of contacting the oxidizing agent is performed at a temperature from 0°C to 100°C.

### (Item 36)

The method of item 35, wherein the step of contacting the oxidizing agent is performed at a temperature of 75°C or higher.

### (Item 37)

The method of item 35, wherein the step of contacting the oxidizing agent is performed at a temperature from 60°C to 90°C.

### (Item 38)

The method of any one of items 1 to 37, wherein the step of contacting the base is performed after the step of contacting the oxidizing agent.

### (Item 39)

The method of any one of items 1 to 38, wherein the step of contacting the base is performed by contacting a base with a reaction mixture produced by the step of contacting the oxidizing agent.

### (Item 40)

The method of any one of items 1 to 39, wherein the oxidizing agent is in the solvent in the step of contacting the base.

### (Item 41)

The method of any one of items 1 to 40, wherein the step of contacting the base is performed in a flow reactor.

### (Item 42)

The method of item 41, wherein an inner diameter of the flow reactor is about 0.1 mm to about 100 mm in the step of contacting the base.

### (Item 43)

The method of item 41 or 42, wherein a residence time of the step of contacting the base is 60 seconds or less.

### (Item 44)

The method of item 43, wherein the residence time is 0.1 seconds to 10 seconds.

### (Item 45)

The method of any one of items 1 to 44, wherein the step of contacting the base is performed at a temperature from 0°C to 100°C.

### (Item 46)

The method of item 45, wherein the step of contacting the base is performed at a temperature from 60°C to 90°C.

### (Item 47)

The method of item 45, wherein the step of contacting the oxidizing agent is performed at a temperature of 75°C or higher.

### (Item 48)

The method of any one of items 1 to 47, wherein a carbon atom, attached with R⁸, R⁹, and OH, is an asymmetric carbon in the compound of formula I.

### (Item 49)

The method of item 48, wherein the compound of formula II is an optically active substance and optical purity is maintained in a reaction between the compound of formula II and the oxidizing agent.

### (Item 50)

The method of any one of items 1 to 49, wherein the compound of formula I is 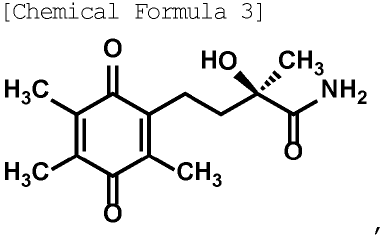 and
the compound of formula II is

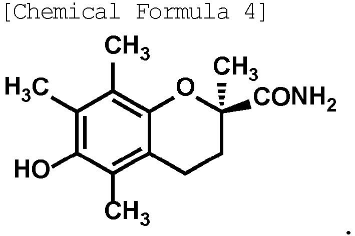

### (Item 51)

The method of item 50, wherein the compound of formula II is manufactured by the step of optically resolving and amidating a compound of formula III:

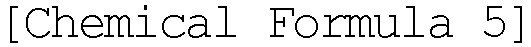

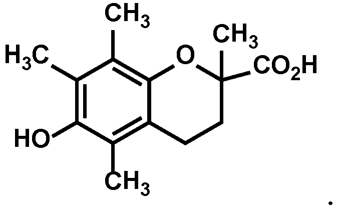

### Advantageous Effects of Invention

The present disclosure provides a method of preparing a compound represented by formula I at a high reaction efficiency with a simple post-processing.

### Brief Description of Drawings

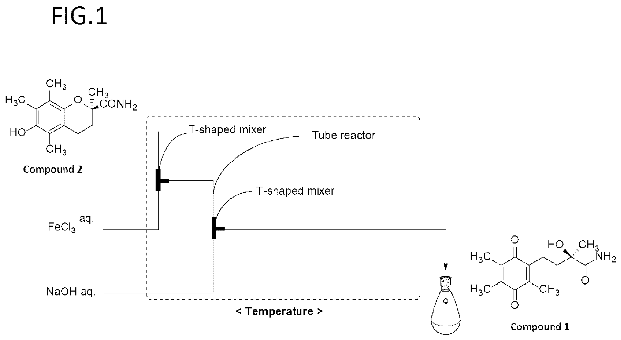

[Figure 1] Figure 1 shows a schematic diagram of an example of the method of the present disclosure using flow synthesis.

### Description of Embodiments

The present disclosure is described hereinafter in more detail. Throughout the entire specification, a singular expression should be understood as encompassing the concept thereof in the plural form, unless specifically noted otherwise. Thus, singular articles (e.g., "a", "an", "the", etc. in the case of English) should also be understood as encompassing the concept thereof in the plural form, unless specifically noted otherwise. Further, the terms used herein should be understood as being used in the meaning that is commonly used in the art, unless specifically noted otherwise. Therefore, unless defined otherwise, all terminologies and scientific technical terms that are used herein have the same meaning as the general understanding of those skilled in the art to which the present disclosure pertains. In case of a contradiction, the present specification (including the definitions) takes precedence.

The present disclosure is further described in detail hereinafter.

The abbreviations used herein have the conventional meaning within the scope of the art unless specifically noted otherwise.

The term "about" for a value or parameter herein includes variations about the value or parameter itself. Unless specifically noted otherwise, "about X" for example includes "X" itself as well as values with an acceptable error of ± 10% therefrom.

As used herein, "Trolox" indicates 6-hydroxy-2,5,7,8-tetramethylchroman-2-carboxylic acid. R form is referred to as R Trolox, and S form is referred to as S Trolox. Trolox can be prepared by a synthesis method that is well known to those skilled in the art, such as the methods described in US Patent No. 3,947,473, US Patent No. 4,003,919, and US Patent No. 4,026,907.

As used herein, "oxidizing agent" refers to a compound that releases an oxygen atom. Examples thereof include, but are not limited to, oxygen, hydrogen peroxide, periodic acid, potassium permanganate, iron(III), etc. Electro-oxidation can also be performed.

As used herein, "base" refers to a substance that receives a proton. Examples thereof include, but are not limited to, sodium hydroxide, potassium hydroxide, calcium hydroxide, barium hydroxide, potassium carbonate, sodium carbonate, sodium hydrogen carbonate, triethylamine, diisopropylethylamine, and ammonia water.

As used herein, "polar aprotic solvent" refers to a polar solvent lacking acidic hydrogen. Examples thereof include, but are not limited to, tetrahydrofuran, 2-methyl-tetrahydrofuran, acetone, acetonitrile, N-methylpyrrolidone, dimethylsulfoxide, etc. "Polar protic solvent" refers to a polar solvent having acidic hydrogen.

As used herein, "residence time" refers to the time required for a fluid comprising a reaction system substance to pass through a portion of a reactor in a flow reaction system.

The number of substituents in a group defined as "optionally substituted" or "substituted" is not particularly limited, as long as they are substitutable. The description of each group is also applicable when the group is a substituent or a part of another group, unless specifically noted otherwise.

A substituent in "optionally substituted" is selected from substituent group α that consists of the following. The substituent is optionally substituted with 1 to 5 of the same or different substituents. While not particularly limited by the type of substituent, if an atom to which the substituent attaches is an oxygen atom, a nitrogen atom, or a sulfur atom, the substituent is limited to the following substituents that attaches to a carbon atom.

### Substituent group α includes

1) a halogen atom
2) a hydroxyl group
3) a carboxyl group
4) a cyano group
5) a C₁₋₆ alkyl group
6) a C₂₋₆ alkenyl group
7) a C₂₋₆ alkynyl group
8) a C₁₋₆ alkoxy group
9) a C₁₋₆ alkylthio group
10) a C₁₋₆ alkylcarbonyl group
11) a C₁₋₆ alkylsulfonyl group
   (wherein each substituent from 5) to 11) is optionally substituted with 1 to 5 of the same or different substituents selected from substituent group β)
12) a C₃₋₁₀ alicyclic group
13) a C₃₋₁₀ alicyclic oxy group
14) a C₆₋₁₀ aryloxy group
15) a 5- or 6-membered heteroaryloxy group
16) a 4- to 10-membered non-aryl heterocyclyl oxy group
17) a C₃₋₁₀ alicyclic thio group
18) a C₆₋₁₀ arylthio group
19) a 5- or 6-membered heteroarylthio group
20) a 4- to 10-membered non-aryl heterocyclyl thio group
21) C₆₋₁₀ aryl
22) 5- or 6-membered heteroaryl
23) a 4- to 10-membered non-aryl heterocycle
24) a C₃₋₁₀ alicyclic carbonyl group
25) a C₆₋₁₀ arylcarbonyl group
26) a 5- or 6-membered heteroarylcarbonyl group
27) a 4- to 10-membered non-aryl heterocyclyl carbonyl group
28) a C₃₋₁₀ alicyclic sulfonyl group
29) a C₆₋₁₀ arylsulfonyl group
30) a 5- or 6-membered heteroarylsulfonyl group
31) a 4- to 10-membered non-aryl heterocyclyl sulfonyl group
   (wherein each substituent from 12) to 31) is optionally substituted with 1 to 5 of substituent group β or 1) a C₁₋₆ alkyl group)
32) -NR^{10a}R^{11a}
33) -SO₂-NR^{10b}R^{11b}
34) -NR^{10c}-C(=O)R^{11c}
35) -NR^{10d}-C(=O)OR^{11d}
36) -NR^{12a}-C(=O)NR^{10e}R^{11e}
37) -NR^{10f}-C(=S)R^{11f}
38) -NR^{10g}-C(=S)OR^{11g},
39) -NR^{12b}-C(=S)NR^{10h}R^{11h}
40) -NR¹⁰ⁱ-SO₂-R¹¹ⁱ
41) -NR^{12c}-SO₂-NR^{10j}R^{11j}
42) -C(=O)OR^{10k}
43) -C(=O)NR^{10l}R^{11k}
44) -C(=O)NR^{10m}OR^{11l}
45) -C(=O)NR^{12d}-NR¹⁰ⁿR^{11m}
46) -C(=S)OR^{10o}
47) -C(=S)NR^{10p}R¹¹ⁿ
48) -C(=S)NR^{10q}OR^{11o}
49) -C(=S)NR^{12e}-NR^{10r}R^{11p}
50) -C(=NR^{13a})R^{10s}
51) -C(=NR^{13b})CHO
52) -C(=NR^{13c})NR^{10t}R^{11q}
53) -C(=NR^{13d})NR^{12f}-NR^{10u}R^{11r}
54) -NR^{17c}-C(=NR^{13k})R^{17d}
55) -NR^{12g}-C(=NR^{13e})-NR^{10v}R^{11s}
56) -NR¹⁴-C(=NR^{13f})NR^{12h}-NR^{10w}R^{11t}
57) -OC(=O)R^{10x}
58) -OC(=O)OR^{10y}
59) -OC(=O)NR^{10z1}R^{11u}
60) -NR¹²ⁱ-NR^{10z2}R^{11v}
61) -NR^{10z3}OR^{11w}
62) -C(=N-OR^{13a})R^{10s}
63) -C(=N-OR^{13b})CHO
64) -C(=N-OR^{13c})NR^{10t}R^{11q}
65) -C(=N-OR^{13d})NR^{12f}-NR^{10u}R^{11r} and
66) -C(=O)H,
substituent group β is a group consisting of
1) a halogen atom,
2) a hydroxyl group,
3) a carboxyl group,
4) a cyano group,
5) a C₃₋₁₀ alicyclic group,
6) a C₁₋₆ alkoxy group,
7) a C₃₋₁₀ alicyclic oxy group,
8) a C₁₋₆ alkylthio group,
9) a 5- or 6-membered heteroarylthio group,
10) C₆₋₁₀ aryl,
11) 5- or 6-membered heteroaryl,
12) a 4- to 10-membered non-aryl heterocycle,
13) a C₁₋₆ alkylcarbonyl group,
14) a C₃₋₁₀ alicyclic carbonyl group,
15) a C₆₋₁₀ arylcarbonyl group,
16) a 5- or 6-membered heteroarylcarbonyl group,
17) a 4- to 10-membered non-aryl heterocyclyl carbonyl group,
18) -NR^{15a}R^{16a},
19) -SO₂-NR^{15b}R^{16b},
20) -NR^{15c}-C(=O)R^{16c},
21) -NR^{17a}-C(=O)NR^{15d}R^{16d},
22) -C(=O)NR^{15e}R^{16e},
23) -C(=NR^{13g})R^{15f},
24) -C(=NR^{13h})NR^{15g}R^{16f},
25) -NR^{16g}-C(=NR¹³ⁱ)R^{15h},
26) -NR^{17b}-C(=NR^{13j})-NR¹⁵ⁱR^{16h},
27) -C(=N-OR^{13g})R^{15f}, and
28) -C(=N-OR^{13h})NR^{15g}R^{16f}
(wherein each substituent from 5) to 17) in substituent group β is optionally substituted with 1 to 5 substituents selected from the group consisting of a halogen atom, a hydroxyl group, a cyano group, a carboxyl group, and -NR^{18a}R^{18b}),
R^{13a}, R^{13b}, R^{13c}, R^{13d}, R^{13e}, R^{13f}, R^{13g}, R^{13h}, R¹³ⁱ, R^{13j}, and R^{13k} are the same or different, each independently a hydrogen atom, a hydroxyl group, a C₁₋₆ alkyl group, or a C₁₋₆ alkoxy group,
R^{10a}, R^{10b}, R^{10c}, R^{10d}, R^{10e}, R^{10f}, R^{10g}, R^{10h}, R¹⁰ⁱ, R^{10j}, R^{10k}, R^{10l}, R^{10m}, R¹⁰ⁿ, R^{10o}, R^{10p}, R^{10q}, R^{10r}, R^{10s}, R^{10t}, R^{10u}, R^{10v}, R^{10w}, R^{10x}, R^{10y}, R^{10z1}, R^{10z2}, R^{10z3}, R^{11a}, R^{11b}, R^{11c}, R^{11d}, R^{11e}, R^{11f}, R^{11g}, R^{11h}, R¹¹ⁱ, R^{11j}, R^{11k}, R^{11l}, R^{11m}, R¹¹ⁿ, R^{11o}, R^{11p}, R^{11q}, R^{11r}, R^{11s}, R^{11t}, R^{11u}, R^{11v}, R^{11w}, R^{12a}, R^{12b}, R^{12c}, R^{12d}, R^{12e}, R^{12f}, R^{12g}, R^{12h}, R¹²ⁱ, R¹⁴, R^{15a}, R^{15b}, R^{15c}, R^{15d}, R^{15e}, R^{15f}, R^{15g}, R^{15h}, R¹⁵ⁱ, R^{16a}, R^{16b}, R^{16c}, R^{16d}, R^{16e}, R^{16f}, R^{16g}, R^{16h}, R^{17a}, R^{17b}, R^{17c}, and R^{17d} are the same or different, each independently a hydrogen atom or a C₁₋₆ alkyl group (wherein the alkyl group is optionally substituted with 1 to 3 of the same or different substituents selected from a hydroxyl group, a cyano group, a C₁₋₆ alkoxy group, and -NR^{18a}R^{18b}), and
R^{18a} and R^{18b} are the same or different, each independently a hydrogen atom or a C₁₋₆ alkyl group.

Preferred examples of substituents in "optionally substituted" include the following substituents.

Preferred substituent group α includes
1) a halogen atom
2) a hydroxyl group
3) a carboxyl group
4) a cyano group
5) a C₁₋₆ alkyl group
6) a C₁₋₆ alkoxy group
7) a C₁₋₆ alkylthio group
8) a C₁₋₆ alkylcarbonyl group
   (wherein each substituent from 5) to 8) is optionally substituted with 1 to 5 of the same or different substituents selected from substituent group β)
9) a C₃₋₁₀ alicyclic group
10) a C₃₋₁₀ alicyclic oxy group
11) a C₆₋₁₀ aryloxy group
12) a 5- or 6-membered heteroaryloxy group
13) a 4- to 10-membered non-aryl heterocyclyl oxy group
14) a C₃₋₁₀ alicyclic thio group
15) a C₆₋₁₀ arylthio group
16) a 5- or 6-membered heteroarylthio group
17) a 4- to 10-membered non-aryl heterocyclyl thio group
18) C₆₋₁₀ aryl
19) 5- or 6-membered heteroaryl
20) a 4- to 10-membered non-aryl heterocycle
21) a C₃₋₁₀ alicyclic carbonyl group
22) a C₆₋₁₀ arylcarbonyl group
23) a 5- or 6-membered heteroarylcarbonyl group
24) a 4- to 10-membered non-aryl heterocyclyl carbonyl group (wherein each substituent from 9) to 24) is optionally substituted with 1 to 5 of substituent group β or 1) a C₁₋₆ alkyl group)
25) -NR^{10a}R^{11a}
26) -SO₂-NR^{10b}R^{11b}
27) -NR^{10c}-C(=O)R^{11c}
28) -NR^{12a}-_{C}(=O)NR^{10d}R^{11d}
29) -NR^{10e}-SO₂-R^{11e}
30) -NR^{12b}-SO₂-NR^{10f}R^{11f}
31) -C(=O)NR^{10g}R^{11g}
32) -C(=NR^{13a})R^{10h}
33) -C(=NR^{13b})NR¹⁰ⁱR^{11h}
34) -NR^{11f}-C(=NR^{13c})R^{10g}
35) -NR^{12c}-C(=NR^{13d})-NR^{10j}R¹¹ⁱ
36) -C(=N-OR^{13a})R^{10h} and
37) -C(=N-OR^{13b})NR¹⁰ⁱR^{11h}
substituent group β is preferably a group consisting of
1) a halogen atom
2) a hydroxyl group
3) a cyano group
4) a C₃₋₁₀ alicyclic group
5) a C₁₋₆ alkoxy group
6) a C₁₋₆ alkylthio group
7) a 5- or 6-membered heteroarylthio group
8) 5- or 6-membered heteroaryl
9) a 4- to 10-membered non-aryl heterocycle
10) a C₁₋₆ alkylcarbonyl group
11) a C₃₋₁₀ alicyclic carbonyl group
12) a C₆₋₁₀ arylcarbonyl group
13) a 5- or 6-membered heteroarylcarbonyl group
14) a 4- to 10-membered non-aryl heterocyclyl carbonyl group
15) -NR^{15a}R^{16a}
16) -NR^{15b}-C(=O)R^{16b}
17) -NR^{17a}-C(=O)NR^{15c}R^{16c}
18) -C(=O)NR^{15d}R^{16d}
19) -C(=NR^{13e})R^{15e}
20) -C(=NR^{13f})NR^{15f}R^{16e}
21) -NR^{16f}-C(=NR^{13g})R^{15g}
22) -NR^{17b}-C(=NR^{13h})-NR^{15h}R^{16g}
23) -C(=N-OR^{13e})R^{15e} and
24) -C(=N-OR^{13f})NR^{15f}R^{16e}
(wherein each substituent from 4) to 14) in substituent group β is optionally substituted with 1 to 5 substituents selected from the group consisting of a halogen atom, a hydroxyl group, a cyano group, a carboxyl group, and -NR^{18a}R^{18b}),
R^{13a}, R^{13b}, R^{13c}, R^{13d}, R^{13e}, R^{13f}, R^{13g}, and R^{13h} are the same or different, each independently a hydrogen atom, a hydroxyl group, a C₁₋₆ alkyl group, or a C₁₋₆ alkoxy group,
R^{10a}, R^{10b}, R^{10c}, R^{10d}, R^{10e}, R^{10f}, R^{10g}, R^{10h}, R¹⁰ⁱ, R^{10j}, R^{11a}, R^{11b}, R^{11c}, R^{11d}, R^{11e}, R^{11f}, R^{11g}*,* R^{11h}, R¹¹ⁱ, R^{12a}, R^{12b}, R^{12c}, R^{15a}, R^{15b}, R^{15c}, R^{15d}, R^{15e}, R^{15f}, R^{15g}, R^{15h}, R^{16a}, R^{16b}, R^{16c}, R^{16d}, R^{16e}, R^{16f}, R^{16g}, R^{17a}, and R^{17b} are the same or different, each independently a hydrogen atom or a C₁₋₆ alkyl group (wherein the alkyl group is optionally substituted with 1 to 3 of the same or different substituents selected from a hydroxyl group, a cyano group, a C₁₋₆ alkoxy group, and -NR^{18a}R^{18b}), and
R^{18a} and R^{18b} are the same or different, each independently a hydrogen atom or a C₁₋₆ alkyl group.

More preferred examples of substituents in "optionally substituted" include the following substituents.

More preferred substituent group α includes
1) a halogen atom
2) a hydroxyl group
3) a cyano group
4) a C₁₋₆ alkyl group
5) a C₁₋₆ alkoxy group
6) a C₁₋₆ alkylthio group
7) a C₁₋₆ alkylcarbonyl group
   (wherein each substituent from 4) to 7) is optionally substituted with 1 to 5 of the same or different substituents selected from substituent group β)
8) a 5- or 6-membered heteroaryloxy group
9) a 4- to 10-membered non-aryl heterocyclyl oxy group
10) a 5- or 6-membered heteroarylthio group
11) a 4- to 10-membered non-aryl heterocyclyl thio group
12) C₆₋₁₀ aryl
13) 5- or 6-membered heteroaryl
14) a 4- to 10-membered non-aryl heterocycle
   (wherein each substituent from 4) to 14) is optionally substituted with 1 to 5 of substituent group β or 1) a C₁₋₆ alkyl group)
15) -NR^{10a}R^{11a}
16) -NR^{11b}-C(=O)R^{10b}
17) -NR^{12a}-C(=O)NR^{10c}R^{11c}
18) -C(=O)NR^{10d}R^{11d}
19) -C(=NR^{13a})R^{10e}
20) -C(=NR^{13b})NR^{10f}R^{11e}
21) -NR^{11f}-C(=NR^{13c})R^{10g}
22) -NR^{12b}-C(=NR^{13d})-NR^{10h}R^{11g}
23) -C(=N-OR^{13a})R^{10e} and
24) -C(=N-OR^{13b})NR^{10f}R^{11e}
substituent group β is more preferably
1) a halogen atom,
2) a hydroxyl group,
3) a cyano group,
4) -NR^{15a}R^{16a},
5) -NR^{15b}-C(=O)R^{16b},
6) -NR^{17a}-C(=O)NR^{15c}R^{16c},
7) -C(=O)NR^{15d}R^{16d},
8) -C(=NR^{13e})R^{15e},
9) -C(=NR^{13f})NR^{15f}R^{16e},
10) -NR^{16f}-C(=NR^{13g})R^{15g},
11) -NR^{17b}-C(=NR^{13h})-NR^{15h}R^{16g}
12) -C(=N-OR^{13e})R^{15e}, and
13) -C(=N-OR^{13f})NR^{15f}R^{16e}

R^{13a}, R^{13b}, R^{13c}, R^{13d}, R^{13e}, R^{13f}, R^{13g}, and R^{13h} are the same or different, each independently a hydrogen atom, a hydroxyl group, a C₁₋₆ alkyl group, or a C₁₋₆ alkoxy group,
R^{10a}, R^{10b}, R^{10c}, R^{10d}, R^{10e}, R^{10f}, R^{10h}, R^{11a}, R^{11b}, R^{11c}, R^{11d}, R^{11e}, R^{11f}, R^{11g}, R^{12a}, R12b, R15a, R^{15b}, R^{15c}, R^{15d}, R^{15e}, R^{15f}, R^{15g}, R^{15h}, R^{16a}, R^{16b}, R^{16c}, R^{16d}, R^{16e}, R^{16f}, R^{16g}, R^{17a}, and R^{17b} are the same or different, each independently a hydrogen atom or a C₁₋₆ alkyl group (wherein the alkyl group is optionally substituted with 1 to 3 of the same or different substituents selected from a hydroxyl group, a cyano group, a C₁₋₆ alkoxy group, and -NR^{18a}R^{18b}), and
R^{18a} and R^{18b} are the same or different, each independently a hydrogen atom or a C₁₋₆ alkyl group.

"C₁₋₆" means that the number of carbon atoms is 1 to 6. The same applies to other numbers. For example, "C₁₋₄" means that the number of carbon atoms is 1 to 4.

A "heteroatom" refers to an oxygen atom, a nitrogen atom, a sulfur atom, etc.

A "halogen atom" refers to a fluorine atom, chlorine atom, bromine atom, or iodine atom, in particular, a fluorine atom and chlorine atom are preferable. A "halogen atom" is also referred to as "halogen".

"C₁₋₆ alkyl" or "C₁₋₆ alkyl group" refers to a linear or branched saturated hydrocarbon group with 1 to 6 carbon atoms. A C₁₋₆ alkyl group is preferably a "C₁₋₄ alkyl group", and more preferably a "C₁₋₃ alkyl group". Specific examples of "C₁₋₃ alkyl group" include methyl, ethyl, propyl, 1-methylethyl, etc. Specific examples of "C₁₋₄ alkyl group" include, in addition to the specific examples specified for the "C₁₋₃ alkyl group" described above, butyl, 1,1-dimethylethyl, 1-methylpropyl, 2-methylpropyl, etc. Specific examples of "C₁₋₆ alkyl group" include, in addition to the specific examples specified for the "C₁₋₄ alkyl group" described above, pentyl, 1,1-dimethylpropyl, 1,2-dimethylpropyl, 1-methylbutyl, 2-methylbutyl, 4-methylpentyl, 3-methylpentyl, 2-methylpentyl, 1-methylpentyl, hexyl, etc.

"C₂₋₆ alkenyl" or "C₂₋₆ alkenyl group" refers to a linear or branched unsaturated hydrocarbon group with 2 to 6 carbon atoms, comprising one or more carbon-carbon double bonds. "C₂₋₆ alkenyl group" is preferably a "C₂₋₄ alkenyl group". Specific examples of "C₂₋₆ alkenyl group" include, but are not limited to, a vinyl group, 1-propylenyl group, 2-propylenyl group, 1-butenyl group, 2-butenyl group, 3-butenyl group, 2-methyl-1-propylenyl group, 2-methyl-2-propylenyl group, etc.

"C₂₋₆ alkynyl" or "C₂₋₆ alkynyl group" refers to a linear or branched unsaturated aliphatic hydrocarbon group having one or more triple bonds. "C₂₋₆ alkynyl group" is preferably a "C₂₋₄ alkynyl group". Specific examples thereof include, but are not limited to, an ethynyl group, 1-propynyl group, 2-propynyl group, 1-butynyl group, 1-methyl-2-propynyl group, 3-butynyl group, 1-pentynyl group, 1-hexynyl group, etc.

"C₃₋₂₀ alicyclic group" refers to a monocyclic or bicyclic non-aromatic hydrocarbon ring group with 3 to 20 carbon atoms, including those with a partially unsaturated bond, those with a partially crosslinked structure, those that have a partially spiro form, and those having one or more carbonyl structures. "Alicyclic group" encompasses cycloalkyl groups, cycloalkenyl groups, and cycloalkynyl groups. "C₃₋₂₀ alicyclic group" is preferably a "C₃₋₁₀ alicyclic group", and more preferably a "C₃₋₇ alicyclic group". Specific examples of "C₃₋₇ alicyclic group" include cyclopropyl, cyclobutyl, cyclopentyl, cyclohexyl, cycloheptyl, etc. Specific examples of "C₃₋₁₀ alicyclic group" include, in addition to the specific examples specified for the "C₃₋₇ alicyclic group" described above, cycloheptyl, cyclooctyl, cyclononyl, cyclodecyl, adamantyl, etc.

Specific examples of "C₃₋₂₀ alicyclic group" with a partially crosslinked structure include, but are not limited to, those with a structure shown below, etc.

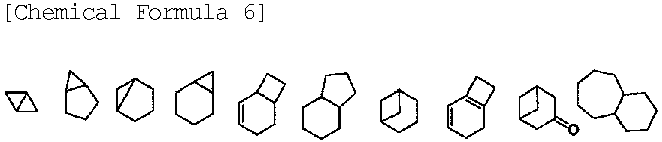

"C₃₋₂₀ alicyclic group" also encompasses compounds fused to an aromatic ring. Specific examples thereof include the groups represented by the following, etc.

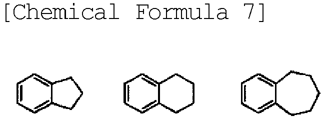

"C₃₋₁₀ alicyclic group" refers to the "C₃₋₂₀ alicyclic group" described above wherein the "C₃₋₁₀ alicyclic group" is a monovalent group.

"C₆₋₁₀ aryl" refers to a monocyclic or bicyclic aromatic hydrocarbon group with 6 to 10 carbon atoms. "C₆₋₁₀ aryl" may be fused to the "alicyclic group" or "non-aryl heterocycle" described above at any possible position. Specific examples of "C₆₋₁₀ aryl" include phenyl, 1-naphthyl, 2-naphthyl, etc. Preferred examples of "C₆₋₁₀ aryl" include phenyl. Specific examples of the fused ring structure include the groups represented by the following, etc.

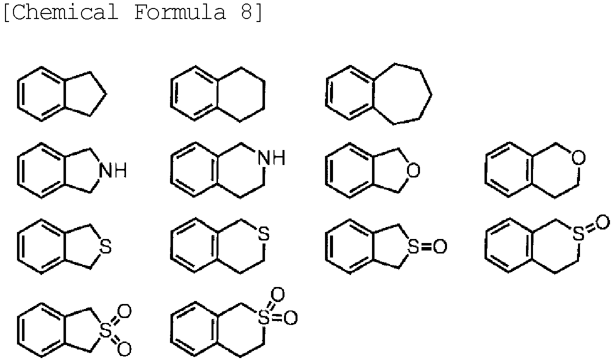

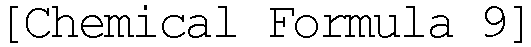

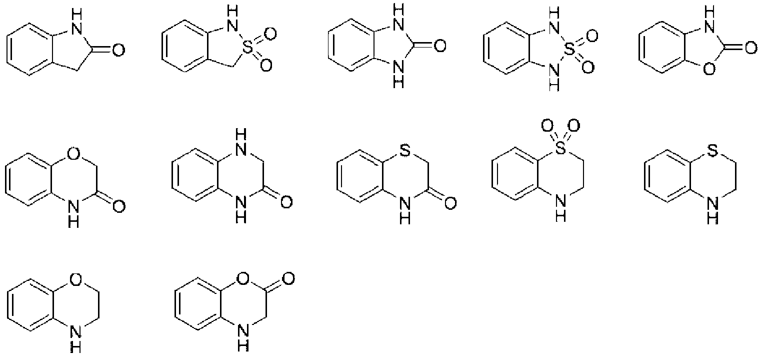

"6- to 10-membered heteroaryl" refers to a monocyclic or bicyclic aromatic heterocyclic group comprised of 6 to 10 atoms, comprising 1 to 4 atoms independently selected from the group consisting of a nitrogen atom, an oxygen atom, and a sulfur atom. "6- to 10-membered heteroaryl" may be fused to the "alicyclic group" or "non-aryl heterocycle" described above at any possible position. "6- to 10-membered heteroaryl" is preferably "6-membered heteroaryl", more preferably pyridyl, pyrazyl, pyrimidyl, or pyridazinyl, and still more preferably pyridyl or pyrimidyl. Specific examples of "6-membered heteroaryl" include pyridyl, pyrazinyl, pyrimidinyl, and pyridazinyl. Specific examples of "6- to 10-membered heteroaryl" include, in addition to the specific examples specified for the "6-membered heteroaryl" described above, quinoxalyl, triazolopyridyl, etc.

Specific examples of "9- or 10-membered heteroaryl" include, but are not limited to, compounds with the structures described below, etc.

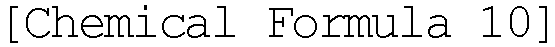

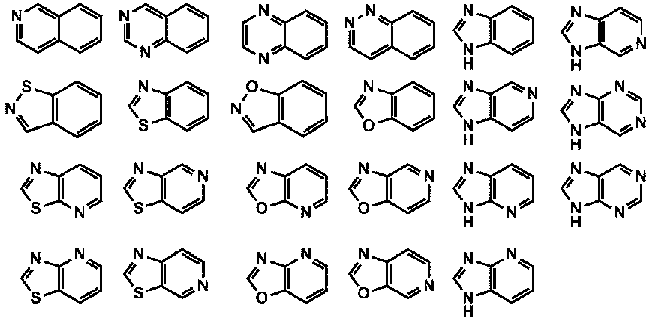

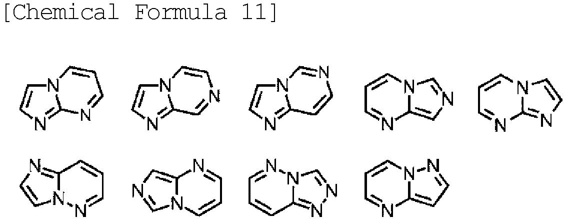

Specific examples of "5-membered heteroaryl" include, but are not limited to, thiophene, pyrrole, thiazole, isothiazole, pyrazole, imidazole, furan, oxazole, isoxazole, oxadiazole, thiadiazole, triazole, tetrazole, etc. 5-membered heteroaryl is preferably pyrazole, imidazole, oxazole, triazole, tetrazole, or thiadiazole, and more preferably imidazole or thiadiazole.

Specific examples of "5- or 6-membered heteroaryl" include the specific examples for the "5-membered heteroaryl" and "6-membered heteroaryl" described above. The "5- or 6-membered heteroaryl" or "5- to 10-membered heteroaryl" described above may form a fused ring structure with a C₅₋₁₀ alicyclic group, or a fused ring structure with a 5- to 10-membered non-aryl heterocycle. Specific examples thereof include the groups represented by the following, etc.

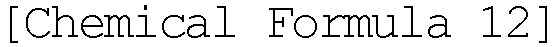

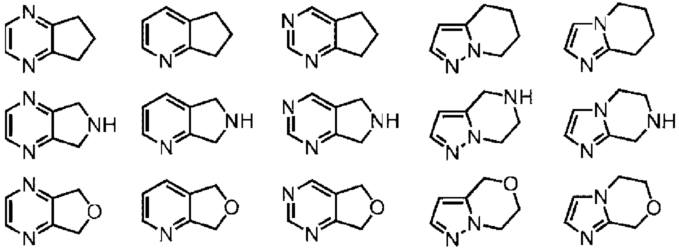

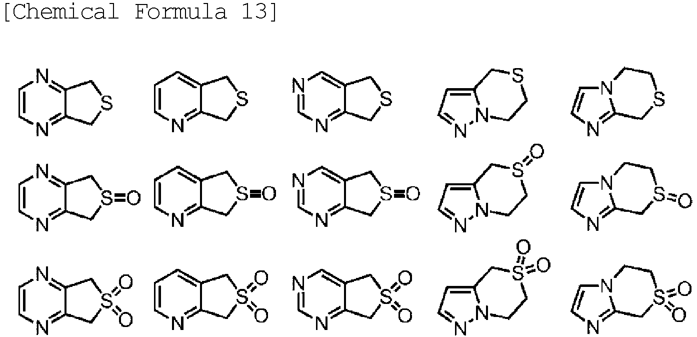

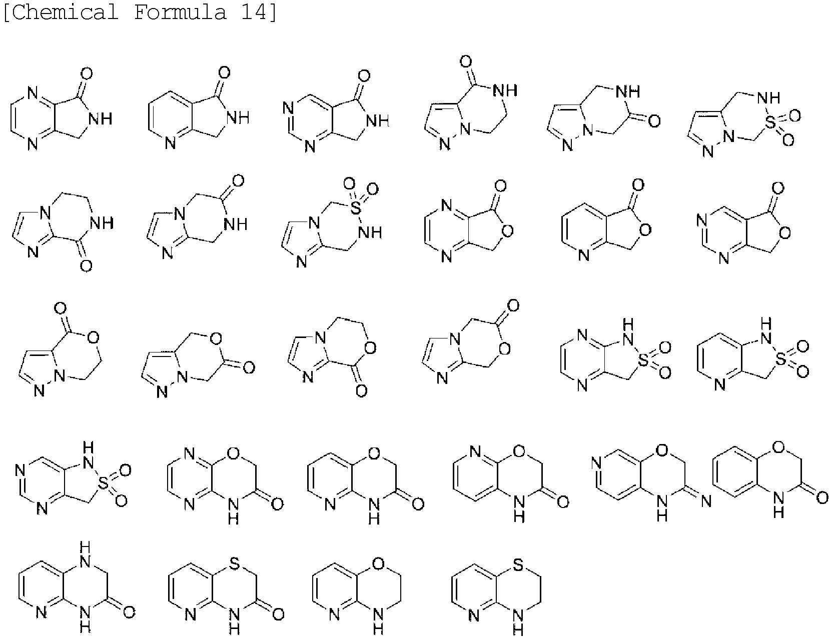

"4- to 20-membered non-aryl heterocyclic group" refers to a monocyclic or bicyclic non-aromatic heterocycle comprised of 4 to 20 atoms, comprising 1 to 2 of the same or different heteroatoms independently selected from the group consisting of a nitrogen atom, an oxygen atom, and a sulfur atom in addition to carbon atoms, including those with a partially unsaturated bond, those with a partially crosslinked structure, and those that have a partially spiro form. "4- to 20-membered non-aryl heterocyclic group" is preferably "4- to 6-membered non-aryl heterocyclic group". Specific examples of "4- to 6-membered non-aryl heterocyclic group" include azetidinyl, pyrrolidinyl, piperidyl, piperazinyl, morpholinyl, tetrahydrofuranyl, tetrahydropyranyl, etc. In particular, azetidinyl, pyrrolidinyl, piperidyl, morpholinyl, and oxetanyl are preferable. A non-aryl heterocycle may form a fused ring with aryl or heteroaryl. Non-aryl heterocycles also encompass those that are fused with, for example, C₆₋₁₀ aryl or 5- or 6-membered heteroaryl. Further, the non-aryl heterocycle may be comprised by including one or more carbonyl, thiocarbonyl, sulfinyl, or sulfonyl. The non-aryl heterocycles also encompass, for example, lactam, thiolactam, lactone, thiolactone, cyclic imide, cyclic carbamate, cyclic thiocarbamate, and other cyclic groups. In this regard, oxygen atoms of carbonyl, sulfinyl, and sulfonyl and sulfur atoms of thiocarbonyl are not included in the number of 4 to 20 members (size of ring) or in the number of heteroatoms constituting a ring. Specific examples of "4- to 20-membered non-aryl heterocycle" include, but are not limited to, azetidine, pyrrolidine, piperidine, piperazine, morpholine, homopiperidine, oxetane, tetrahydrofuran, tetrahydropyran, heterocycles with the following structure, etc.

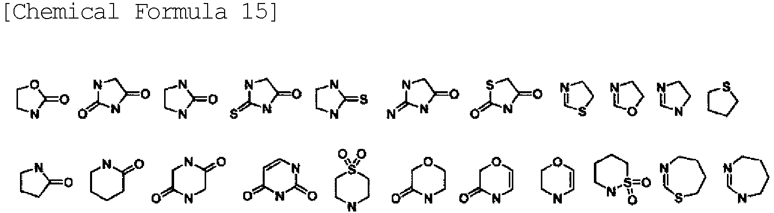

Specific examples of "4- to 20-membered non-aryl heterocycle" with partial crosslinking or spiro structure include, but are not limited to, those with a structure shown below, etc.

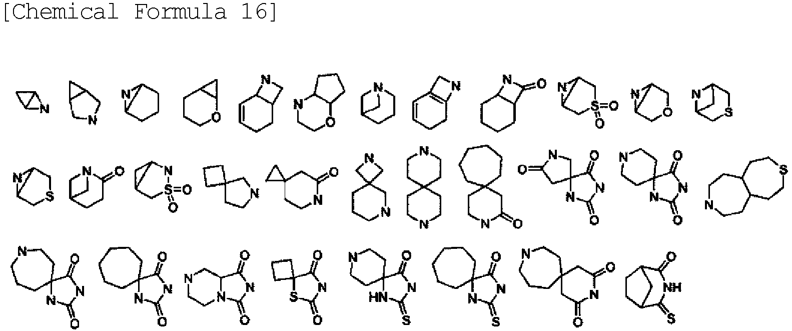

Specific examples of "4-membered non-aryl heterocycle" having a partially unsaturated bond include, but are not limited to, those with a structure shown below, etc. [Chemical Formula 17]

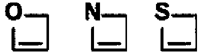

Specific examples of "S-membered non-aryl heterocycle" with a partially unsaturated bond include, but are not limited to, those with a structure shown below, etc.

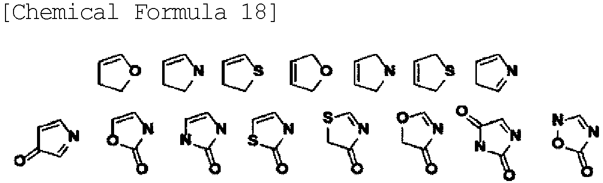

Specific examples of "S-membered non-aryl heterocycle" with a partially crosslinked structure include, but are not limited to, those with a structure shown below, etc. [Chemical Formula 19]

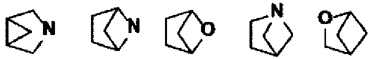

Specific examples of "S-membered non-aryl heterocycle" comprising carbonyl, thiocarbonyl, etc. include, but are not limited to, those with a structure shown below, etc.

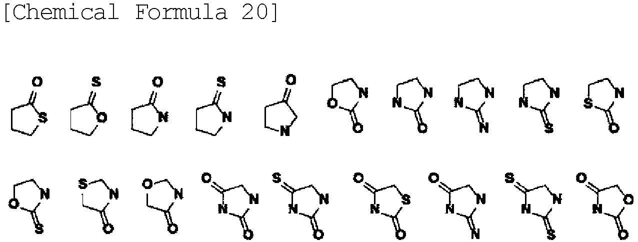

Specific examples of "6-membered non-aryl heterocycle" with a partially unsaturated bond include, but are not limited to, those with a structure shown below, etc.

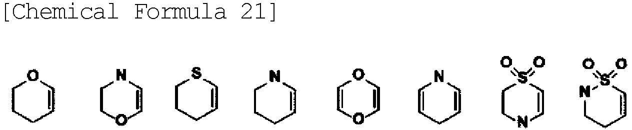

Specific examples of "6-membered non-aryl heterocycle" with a partially crosslinked structure include, but are not limited to, those with a structure shown below, etc.

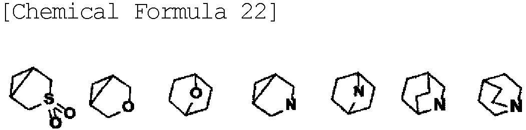

"C₁₋₆ alkoxy" or "C₁₋₆ alkoxy group" refers to "C₁₋₆ alkyloxy", and the "C₁₋₆ alkyl" moiety is defined the same as the "C₁₋₆ alkyl" described above. "C₁₋₆ alkoxy" is preferably "C₁₋₄ alkoxy", and more preferably "C₁₋₃ alkoxy". Specific examples of "C₁₋₃ alkoxy" include methoxy, ethoxy, propoxy, 1-methylethoxy, etc. Specific examples of "C₁₋₄ alkoxy" include, in addition to the specific examples specified for the "C₁₋₃ alkoxy" described above, butoxy, 1,1-dimethylethoxy, 1-methylpropoxy, 2-methylpropoxy, etc. Specific examples of "C₁₋₆ alkoxy" include, in addition to the specific examples specified for the "C₁₋₄ alkoxy" described above, pentyloxy, 1,1-dimethylpropoxy, 1,2-dimethylpropoxy, 1-methylbutoxy, 2-methylbutoxy, 4-methylpentyloxy, 3-methylpentyloxy, 2-methylpentyloxy, 1-methylpentyloxy, hexyloxy, etc.

"C₃₋₆ alicyclic oxy" or "C₃₋₆ alicyclic oxy group" refers to a (C₃₋₆ alicyclic group)-O-group, and the C₃₋₆ alicyclic moiety is defined the same as a C₃₋₆ alicyclic group. "C₃₋₆ alicyclic oxy group" includes "C₃₋₆ cycloalkoxy group". "Cycloalkoxy group" refers to "cycloalkyloxy", and the "cycloalkyl" moiety is defined the same as the "cycloalkyl" described above. Specific examples of "C₃₋₆ alicyclic oxy group" include a cyclopropoxy group, cyclobutoxy group, cyclopentoxy group, cyclohexoxy group, etc.

The C₆₋₁₀ aryl moiety of "C₆₋₁₀ aryloxy group" is defined the same as the C₆₋₁₀ aryl described above. "C₆₋₁₀ aryloxy group" is preferably a "C₆ or C₁₀ aryloxy group". Specific examples of "C₆₋₁₀ aryloxy group" include, but are not limited to, a phenoxy group, 1-naphthyloxy group, 2-naphthyloxy group, etc.

The 5- or 6-membered heteroaryl moiety of "5- or 6-membered heteroaryloxy group" is defined the same as the "S-membered heteroaryl" or "6-membered heteroaryl" described above. Specific examples of "5- or 6-membered heteroaryloxy group" include, but are not limited to, a pyrazoyloxy group, triazoyloxy group, thiazoyloxy group, thiadiazoyloxy group, pyridyloxy group, pyridazoyloxy group, etc.

The 4- to 10-membered non-aryl heterocycle moiety of "4- to 10-membered non-aryl heterocyclyl oxy group" is defined the same as the "4- to 10-membered non-aryl heterocycle" described above. "4- to 10-membered non-aryl heterocyclyl oxy group" is preferably a "4- to 6-membered non-aryl heterocyclyl oxy group". Specific examples of "4-to 10-membered non-aryl heterocyclyl oxy group" include, but are not limited to, a tetrahydrofuranyloxy group, tetrahydropyranyloxy group, azetidinyloxy group, pyrrolidinyloxy group, piperidinyloxy group, etc.

The C₁₋₆ alkyl moiety of "C₁₋₆ alkylthio group" is defined the same as the C₁₋₆ alkyl described above. "C₁₋₆ alkylthio group" is preferably a "C₁₋₄ alkylthio group", and more preferably a "C₁₋₃ alkylthio group". Specific examples of "C₁₋₆ alkylthio group" include, but are not limited to, a methylthio group, ethylthio group, propylthio group, butylthio group, isopropylthio group, isobutylthio group, tert-butylthio group, sec-butylthio group, isopentylthio group, neopentylthio group, tert-pentylthio group, 1,2-dimethylpropylthio group, etc.

"C₃₋₁₀ alicyclic thio" or "C₃₋₁₀ alicyclic thio group" refers to a (C₃₋₁₀ alicyclic group) -S-group, and the C₃₋₁₀ alicyclic moiety is defined the same as the C₃₋₁₀ alicyclic group described above. "C₃₋₁₀ alicyclic thio group" is preferably a "C₃₋₆ alicyclic thio group". Specific examples of "C₃₋₆ alicyclic thio group" include, but are not limited to, a cyclopropylthio group, cyclobutylthio group, cyclopentylthio group, cyclohexylthio group, etc.

The C₆₋₁₀ aryl moiety of "C₆₋₁₀ arylthio" or "C₆₋₁₀ arylthio group" is defined the same as the C₆₋₁₀ aryl described above. "C₆₋₁₀ arylthio group" is preferably a "C₆ or C₁₀ arylthio group". Specific examples of "C₆₋₁₀ aryloxy group" include, but are not limited to, a phenylthio group, 1-naphthylthio group, 2-naphthylthio group, etc.

The 5- or 6-membered heteroaryl moiety of "5- or 6-membered heteroarylthio" or "5- or 6-membered heteroarylthio group" is defined the same as the "S-membered heteroaryl" or "6-membered heteroaryl" described above. Specific examples of "5- or 6-membered heteroarylthio group" include, but are not limited to, a pyrazoylthio group, triazoylthio group, thiazoylthio group, thiadiazoylthio group, pyridylthio group, pyridazoylthio group, etc.

The 4- to 10-membered non-aryl heterocycle moiety of "4- to 10-membered non-aryl heterocyclyl thio" or "4- to 10-membered non-aryl heterocyclyl thio group" is defined the same as the "4- to 10-membered non-aryl heterocycle" described above. "4- to 10-membered non-aryl heterocyclyl thio group" is preferably a "4- to 6-membered non-aryl heterocyclyl thio group". Specific examples of "4- to 10-membered non-aryl heterocyclyl thio group" include, but are not limited to, a tetrahydropyranylthio group, piperidinylthio group, etc.

"C₁₋₆ alkylcarbonyl" or "C₁₋₆ alkylcarbonyl group" refers to a carbonyl group substituted with the "C₁₋₆ alkyl group" described above. "C₁₋₆ alkylcarbonyl group" is preferably a "C₁₋₄ alkylcarbonyl group". Specific examples of "C₁₋₆ alkylcarbonyl group" include, but are not limited to, an acetyl group, propionyl group, butyryl group, etc.

"C₃₋₁₀ alicyclic carbonyl" or "C₃₋₁₀ alicyclic carbonyl group" refers to a carbonyl group substituted with the "C₃₋₁₀ alicyclic group" described above. "C₃₋₁₀ alicyclic carbonyl group" is preferably a "C₃₋₆ alicyclic carbonyl group". Specific examples of "C₃₋₁₀ alicyclic carbonyl group" include, but are not limited to, a cyclopropylcarbonyl group, cyclopentylcarbonyl group, etc.

"C₆₋₁₀ arylcarbonyl" or "C₆₋₁₀ arylcarbonyl group" refers to a carbonyl group substituted with the "C₆₋₁₀ aryl" described above. "C₆₋₁₀ arylcarbonyl group" is preferably a "C₆ or C₁₀ arylcarbonyl group". Specific examples of "C₆₋₁₀ arylcarbonyl group" include, but are not limited to, a benzoyl group, 1-naphthylcarbonyl group, 2-naphthylcarbonyl group, etc.

"5- or 6-membered heteroarylcarbonyl" or "5- or 6-membered heteroarylcarbonyl group" refers to a carbonyl group substituted with the "5- or 6-membered heteroaryl" described above. Specific examples of "5- or 6-membered heteroarylcarbonyl group" include, but are not limited to, a pyrazoylcarbonyl group, triazoylcarbonyl group, thiazoylcarbonyl group, thiadiazoylcarbonyl group, pyridylcarbonyl group, pyridazoylcarbonyl group, etc.

"4- to 10-membered non-aryl heterocyclyl carbonyl" or "4- to 10-membered non-aryl heterocyclyl carbonyl group" refers to a carbonyl group substituted with the "4- to 10-membered non-aryl heterocycle" described above. "4- to 10-membered non-aryl heterocyclyl carbonyl group" is preferably a "4- to 6-membered non-aryl heterocyclyl carbonyl group". Specific examples of "4- to 10-membered non-aryl heterocyclyl carbonyl group" include, but are not limited to, an azetidinylcarbonyl group, pyrrolidinylcarbonyl group, piperidinylcarbonyl group, morpholinylcarbonyl group, etc.

"C₁₋₆ alkylsulfonyl" or "C₁₋₆ alkylsulfonyl group" refers to a sulfonyl group substituted with the "C₁₋₆ alkyl group" described above. "C₁₋₆ alkylsulfonyl group" is preferably a "C₁₋₄ alkylsulfonyl group". Specific examples of "C₁₋₆ alkylsulfonyl group" include, but are not limited to, a methylsulfonyl group, propionylsulfonyl group, butyrylsulfonyl group, etc.

"C₃₋₁₀ alicyclic sulfonyl" or "C₃₋₁₀ alicyclic sulfonyl group" refers to a sulfonyl group substituted with the "C₃₋₁₀ alicyclic group" described above. "C₃₋₁₀ alicyclic sulfonyl group" is preferably a "C₃₋₆ alicyclic sulfonyl group". Specific examples of "C₃₋₁₀ alicyclic sulfonyl group" include, but are not limited to, a cyclopropylsulfonyl group, cyclobutylsulfonyl group, cyclopentylsulfonyl group, cyclohexylsulfonyl group, etc.

"C₆₋₁₀ arylsulfonyl" or "C₆₋₁₀ arylsulfonyl group" refers to a sulfonyl group substituted with the "C₆₋₁₀ aryl" described above. "C₆₋₁₀ arylsulfonyl group" is preferably a "C₆ or C₁₀ arylsulfonyl group". Specific examples of "C₆₋₁₀ arylsulfonyl group" include, but are not limited to, a phenylsulfonyl group, 1-naphthylsulfonyl group, 2-naphthylsulfonyl group, etc.

"5- or 6-membered heteroarylsulfonyl" or "5- or 6-membered heteroarylsulfonyl group" refers to a sulfonyl group substituted with the "5- or 6-membered heteroaryl" described above. Specific examples of "5- or 6-membered heteroarylsulfonyl group" include a pyrazoylsulfonyl group, triazoylsulfonyl group, thiazoylsulfonyl group, thiadiazoylsulfonyl group, pyridylsulfonyl group, pyridazoylsulfonyl group, etc.

As used herein, "optical purity is maintained" indicates that optical purity does not change significantly when optical purity is measured before and after the mentioned reaction, and preferably indicates that the change is 20% ee or less. The change in optical purity when "optical purity is maintained" is, for example, 10% ee or less, 5% ee or less, 3% ee or less, 1% ee or less, or 0.5% ee or less.

### (Preferred embodiments)

Preferred embodiments of the present disclosure are described below. Embodiments described below are provided to facilitate the understanding of the present disclosure. The scope of the present disclosure should not be limited to the following descriptions. Thus, it is apparent that those skilled in the art can make appropriate modifications within the scope of the present disclosure by referring to the descriptions herein. The following embodiments of the present disclosure can be used independently or as a combination thereof.

One embodiment of the present disclosure provides a method of manufacturing a compound of formula I: 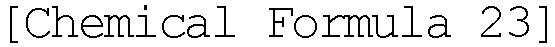 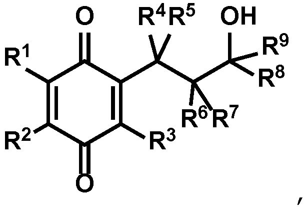 comprising the steps of:
contacting, in a solvent, an oxidizing agent with a compound of formula II: 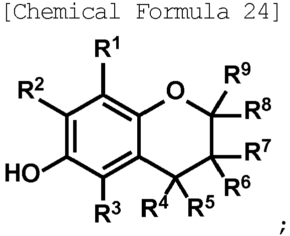 and
contacting a base with the solvent to promote a reaction between the compound of formula II and the oxidizing agent;
wherein
R¹, R², R³, R⁴, R⁵, R⁶, R⁷, R⁸, and R⁹ each independently represent a hydrogen atom, halogen, optionally substituted C₁₋₆ alkyl, optionally substituted C₁₋₆ alkoxy, or -C(=O)NR^{A}R^{B}, and
R^{A} and R^{B} each independently represent a hydrogen atom, optionally substituted C₁₋₆ alkyl, or optionally substituted C₁₋₆ alkoxy.

In the present disclosure, R¹ can be a hydrogen atom or optionally substituted C₁₋₆ alkyl.

In the present disclosure, R¹ can be methyl.

In the present disclosure, R² can be a hydrogen atom or optionally substituted C₁₋₆ alkyl.

In the present disclosure, R² can be methyl.

In the present disclosure, R³ can be a hydrogen atom or optionally substituted C₁₋₆ alkyl.

In the present disclosure, R³ can be methyl.

In the present disclosure, R⁴ can be a hydrogen atom or optionally substituted C₁₋₆ alkyl.

In the present disclosure, R⁴ can be a hydrogen atom.

In the present disclosure, R⁵ can be a hydrogen atom or optionally substituted C₁₋₆ alkyl.

In the present disclosure, R⁵ can be a hydrogen atom.

In the present disclosure, R⁶ can be a hydrogen atom or optionally substituted C₁₋₆ alkyl.

In the present disclosure, R⁶ can be a hydrogen atom.

In the present disclosure, R⁷ can be a hydrogen atom or optionally substituted C₁₋₆ alkyl.

In the present disclosure, R⁷ can be a hydrogen atom.

In the present disclosure, R⁸ can be a hydrogen atom, optionally substituted C₁₋₆ alkyl, or -C(=O)NR^{A}R^{B}.

In the present disclosure, R⁸ can be -C(=O)NR^{A}R^{B}.

In the present disclosure, R⁹ can be a hydrogen atom or optionally substituted C₁₋₆ alkyl.

In the present disclosure, R⁹ can be methyl.

In the present disclosure, R^{A} and R^{B} each can represent a hydrogen atom.

In the present disclosure, the solvent can be a polar aprotic solvent.

In the present disclosure, the solvent can be a polar protic solvent.

In the present disclosure, the solvent can be selected from the group consisting of isopropyl acetate, ethyl acetate, tetrahydrofuran, 2-methyl-tetrahydrofuran, acetone, toluene, anisole, methyl tert-butyl ether, cyclopentyl methyl ether, methanol, ethanol, 2-propanol, 4-methyltetrahydropyran, acetonitrile, N-methylpyrrolidone, and dimethylsulfoxide.

In the present disclosure, the solvent can be dimethylsulfoxide.

In the present disclosure, the oxidizing agent can comprise a compound comprising iron.

In the present disclosure, the oxidizing agent can be iron(III) chloride or iron(III) nitrate.

In the present disclosure, the oxidizing agent can be iron(III) chloride hexahydrate.

In the present disclosure, the base can be selected from the group consisting of sodium hydroxide, potassium hydroxide, calcium hydroxide, barium hydroxide, potassium carbonate, sodium carbonate, sodium hydrogen carbonate, triethylamine, diisopropylethylamine, and ammonia water.

In the present disclosure, the base can be selected from the group consisting of sodium hydroxide, potassium carbonate, sodium carbonate, sodium hydrogen carbonate, triethylamine, and ammonia water.

In the present disclosure, the base can be sodium hydroxide.

In the present disclosure, the step of contacting the oxidizing agent can be performed in a flow reactor.

In the present disclosure, an inner diameter of the flow reactor can be about 0.1 mm to about 100 mm in the step of contacting the oxidizing agent. The inner diameter of the flow reactor in the step of contacting the oxidizing agent can be about 0.1 mm or greater, about 0.2 mm or greater, about 0.3 mm or greater, about 0.4 mm or greater, about 0.5 mm or greater, about 0.6 mm or greater, about 0.7 mm or greater, about 0.8 mm or greater, about 0.9 mm or greater, about 1.0 mm or greater, about 1.1 mm or greater, about 1.2 mm or greater, about 1.3 mm or greater, about 1.4 mm or greater, about 1.5 mm or greater, about 1.6 mm or greater, about 1.7 mm or greater, about 1.8 mm or greater, about 1.9 mm or greater, about 2.0 mm or greater, about 2.5 mm or greater, about 3.0 mm or greater, about 3.5 mm or greater, about 4.0 mm or greater, about 4.5 mm or greater, about 5.0 mm or greater, about 5.5 mm or greater, about 6.0 mm or greater, about 6.5 mm or greater, about 7.0 mm or greater, about 7.5 mm or greater, about 8.0 mm or greater, about 8.5 mm or greater, about 9.0 mm or greater, about 9.5 mm or greater, about 10 mm or greater, about 15 mm or greater, about 20 mm or greater, about 25 mm or greater, about 30 mm or greater, about 35 mm or greater, about 40 mm or greater, about 45 mm or greater, about 50 mm or greater, about 55 mm or greater, about 60 mm or greater, about 65 mm or greater, about 70 mm or greater, about 75 mm or greater, about 80 mm or greater, about 85 mm or greater, about 90 mm or greater, or about 95 mm or greater, or about 100 mm or less, about 95 mm or less, about 90 mm or less, about 85 mm or less, about 80 mm or less, about 75 mm or less, about 70 mm or less, about 65 mm or less, about 60 mm or less, about 55 mm or less, about 50 mm or less, about 45 mm or less, about 40 mm or less, about 35 mm or less, about 30 mm or less, about 25 mm or less, about 20 mm or less, about 15 mm or less, about 10 mm or less, about 9.5 mm or less, about 9.0 mm or less, about 8.5 mm or less, about 8.0 mm or less, about 7.5 mm or less, about 7.0 mm or less, about 6.5 mm or less, about 6.0 mm or less, about 5.5 mm or less, about 5.0 mm or less, about 4.5 mm or less, about 4.0 mm or less, about 3.5 mm or less, about 3.0 mm or less, about 2.5 mm or less, about 2.0 mm or less, about 1.9 mm or less, about 1.8 mm or less, about 1.7 mm or less, about 1.6 mm or less, about 1.5 mm or less, about 1.4 mm or less, about 1.3 mm or less, about 1.2 mm or less, about 1.1 mm or less, about 1.0 mm or less, about 0.9 mm or less, about 0.8 mm or less, about 0.7 mm or less, about 0.6 mm or less, about 0.5 mm or less, about 0.4 mm or less, about 0.3 mm or less, or about 0.2 mm or less, preferably 0.5 mm or greater and about 20 mm or less, more preferably about 0.7 mm or greater and about 15 mm or less, still more preferably about 0.5 mm or greater and about 10 mm or less, and especially preferably about 1.0 mm or greater and about 10 mm or less.

In the present disclosure, a residence time of the step of contacting the oxidizing agent can be 60 seconds or less. The residence time of the step of contacting the oxidizing agent can be 60 seconds or less, 50 seconds or less, 40 seconds or less, 30 seconds or less, 20 seconds or less, 10 seconds or less, 5 seconds or less, 4 seconds or less, 3 seconds or less, 2 seconds or less, or 1 second or less.

In the present disclosure, the residence time of the step of contacting the oxidizing agent can be 0.1 seconds to 10 seconds. The residence time of the step of contacting the oxidizing agent can be 0.1 seconds to 5 seconds.

In the present disclosure, the step of contacting the oxidizing agent can be performed at a temperature from 0°C to 100°C. The step of contacting the oxidizing agent can be performed at a temperature of 0°C or higher, 5°C or higher, 10°C or higher, 15°C or higher, 20°C or higher, 25°C or higher, 30°C or higher, 35°C or higher, 40°C or higher, 50°C or higher, 55°C or higher, 60°C or higher, 65°C or higher, 70°C or higher, 75°C or higher, 80°C or higher, 85°C or higher, 90°C or higher, or 95°C or higher, or 100°C or lower, 95°C or lower, 90°C or lower, 85°C or lower, 80°C or lower, 75°C or lower, 70°C or lower, 65°C or lower, 60°C or lower, 55°C or lower, 50°C or lower, 45°C or lower, 40°C or lower, 35°C or lower, 30°C or lower, 25°C or lower, 20°C or lower, 15°C or lower, 10°C or lower, or 5°C or lower, preferably 30°C or greater and 100°C or lower, more preferably 65°C or higher and 95°C or lower, and still more preferably 60°C or higher and 90°C or lower.

In the present disclosure, the step of contacting the oxidizing agent can be performed at a temperature of 75°C or higher.

In the present disclosure, the step of contacting the base can be performed after the step of contacting the oxidizing agent.

In the present disclosure, the step of contacting the base can be performed by contacting a base with a reaction mixture produced by the step of contacting the oxidizing agent.

In the present disclosure, the oxidizing agent can be in the solvent in the step of contacting the base.

In the present disclosure, the step of contacting the base can be performed in a flow reactor.

In the present disclosure, an inner diameter of the flow reactor can be about 0.1 mm to about 100 mm in the step of contacting the base. The inner diameter of the flow reactor in the step of contacting the base can be about 0.1 mm or greater, about 0.2 mm or greater, about 0.3 mm or greater, about 0.4 mm or greater, about 0.5 mm or greater, about 0.6 mm or greater, about 0.7 mm or greater, about 0.8 mm or greater, about 0.9 mm or greater, about 1.0 mm or greater, about 1.1 mm or greater, about 1.2 mm or greater, about 1.3 mm or greater, about 1.4 mm or greater, about 1.5 mm or greater, about 1.6 mm or greater, about 1.7 mm or greater, about 1.8 mm or greater, about 1.9 mm or greater, about 2.0 mm or greater, about 2.5 mm or greater, about 3.0 mm or greater, about 3.5 mm or greater, about 4.0 mm or greater, about 4.5 mm or greater, about 5.0 mm or greater, about 5.5 mm or greater, about 6.0 mm or greater, about 6.5 mm or greater, about 7.0 mm or greater, about 7.5 mm or greater, about 8.0 mm or greater, about 8.5 mm or greater, about 9.0 mm or greater, about 9.5 mm or greater, about 10 mm or greater, about 15 mm or greater, about 20 mm or greater, about 25 mm or greater, about 30 mm or greater, about 35 mm or greater, about 40 mm or greater, about 45 mm or greater, about 50 mm or greater, about 55 mm or greater, about 60 mm or greater, about 65 mm or greater, about 70 mm or greater, about 75 mm or greater, about 80 mm or greater, about 85 mm or greater, about 90 mm or greater, or about 95 mm or greater, or about 100 mm or less, about 95 mm or less, about 90 mm or less, about 85 mm or less, about 80 mm or less, about 75 mm or less, about 70 mm or less, about 65 mm or less, about 60 mm or less, about 55 mm or less, about 50 mm or less, about 45 mm or less, about 40 mm or less, about 35 mm or less, about 30 mm or less, about 25 mm or less, about 20 mm or less, about 15 mm or less, about 10 mm or less, about 9.5 mm or less, about 9.0 mm or less, about 8.5 mm or less, about 8.0 mm or less, about 7.5 mm or less, about 7.0 mm or less, about 6.5 mm or less, about 6.0 mm or less, about 5.5 mm or less, about 5.0 mm or less, about 4.5 mm or less, about 4.0 mm or less, about 3.5 mm or less, about 3.0 mm or less, about 2.5 mm or less, about 2.0 mm or less, about 1.9 mm or less, about 1.8 mm or less, about 1.7 mm or less, about 1.6 mm or less, about 1.5 mm or less, about 1.4 mm or less, about 1.3 mm or less, about 1.2 mm or less, about 1.1 mm or less, about 1.0 mm or less, about 0.9 mm or less, about 0.8 mm or less, about 0.7 mm or less, about 0.6 mm or less, about 0.5 mm or less, about 0.4 mm or less, about 0.3 mm or less, or about 0.2 mm or less, preferably about 0.5 mm or greater and about 20 mm or less, more preferably about 0.7 mm or greater and about 15 mm or less, still more preferably about 0.5 mm or greater and about 10 mm or less, and especially preferably about 1.0 mm or greater and about 10 mm or less.

In the present disclosure, a residence time of the step of contacting the base can be 60 seconds or less. A residence time of the step of contacting the base can be 60 seconds or less, 50 seconds or less, 40 seconds or less, 30 seconds or less, 20 seconds or less, 10 seconds or less, 5 seconds or less, 4 seconds or less, 3 seconds or less, 2 seconds or less, or 1 second or less.

In the present disclosure, the residence time of the step of contacting the base can be 0.1 seconds to 10 seconds. The residence time of the step of contacting the base can be 0.1 seconds to 5 seconds.

In the present disclosure, the step of contacting the base can be performed at a temperature from 0°C to 100°C. The step of contacting the base can be performed at a temperature of 0°C or higher, 5°C or higher, 10°C or higher, 15°C or higher, 20°C or higher, 25°C or higher, 30°C or higher, 35°C or higher, 40°C or higher, 50°C or higher, 55°C or higher, 60°C or higher, 65°C or higher, 70°C or higher, 75°C or higher, 80°C or higher, 85°C or higher, 90°C or higher, or 95°C or higher, or 100°C or lower, 95°C or lower, 90°C or lower, 85°C or lower, 80°C or lower, 75°C or lower, 70°C or lower, 65°C or lower, 60°C or lower, 55°C or lower, 50°C or lower, 45°C or lower, 40°C or lower, 35°C or lower, 30°C or lower, 25°C or lower, 20°C or lower, 15°C or lower, 10°C or lower, or 5°C or lower, preferably 30°C or higher and 100°C or lower, more preferably 65°C or higher and 95°C or lower, and still more preferably 60°C or higher and 90°C or lower.

In the present disclosure, the step of contacting the oxidizing agent can be performed at a temperature of 75°C or higher.

In the present disclosure, a carbon atom, attached with R⁸, R⁹, and OH, are attached can be asymmetric carbon in the compound of formula I.

In the present disclosure, the compound of formula II can be an optically active substance and optical purity can be maintained in a reaction between the compound of formula II and the oxidizing agent.

In the present disclosure, the compound of formula I can be 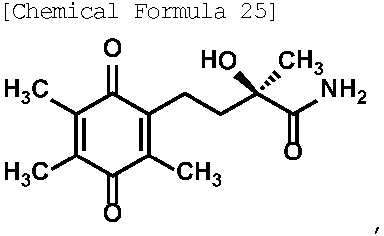 and
the compound of formula II can be

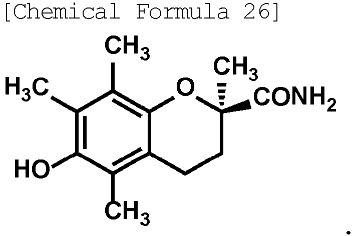

In the present disclosure, the compound of formula II can be manufactured by the step of optically resolving and amidating a compound of formula III: 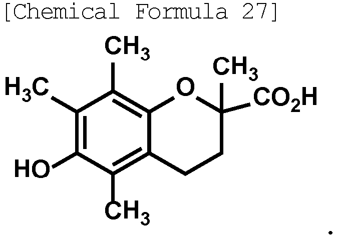 Optical resolution of a compound of formula III can be performed by the method described in WO 2021/167095.

### (Manufacturing method)

A compound of formula I is manufactured by contacting an oxidizing agent with a compound of formula II in the present disclosure. The method of the present disclosure comprises the step of contacting a base with the solvent to promote a reaction between the compound of formula II and the oxidizing agent.

In one embodiment of the present disclosure, an oxidizing agent can comprise a compound comprising iron. When iron(III) chloride was used as an oxidizing agent, the endpoint of a reaction could not be reached by a single administration of a reagent, but the reaction rate dramatically improved when a base was added. Meanwhile, addition of a base resulted in the generation and precipitation of iron(III) hydroxide, so that a risk of delaying the reaction was found. If a hydroxide ion and iron(III) chloride in the system react and precipitate as iron hydroxide, the reaction endpoint is directly reached (risk of delay in reaction upon addition of a base). Iron(III) hydroxide is poorly soluble in water or solvent and is difficult to retrieve after precipitation. It is preferable to strictly control the order and timing of adding a base for more efficient progression of a reaction.

In one embodiment of the present disclosure, a compound of formula I can be manufactured by flow synthesis.

Flow synthesis (also known as flow chemistry) refers to a synthesis technology or manufacturing technology for performing a chemical reaction in a "flow". Use of an unstable raw material or intermediate and a hazardous reactions are not suitable for a batch reaction, so that it is essential to consider scale-up. Meanwhile, flow synthesis can be performed in an optimal reaction time, can precisely manage the added amount, and can provide an efficient reaction space. Thus, flow synthesis is compatible with various reactions by connecting equipment and increasing/decreasing the number of flow manufacturing equipment. A reaction space can be readily designed by combining modules of manufacturing equipment.

In the present disclosure, a reaction can be performed at a suitable timing, accurately, and reproducibly by applying a flow synthesis technology.

In one embodiment of the present disclosure, a compound of formula I is manufactured by an oxidation reaction using iron(III) chloride in a flow reactor. A reaction in a flow reactor did not require separation of solutions, which was required in batch reactions. It was difficult to faithfully reproduce the process materialized by a flow reaction in a batch reactor. Scale-up was difficult in a batch reaction due to difficulty in stirring. When iron(III) chloride is used instead of iron nitrate, nitrosamine is not produced from the reaction mechanism. High-speed mixture and precise flow rate control of a flow synthesis technology resulted in successfully improving reaction efficiency and simplifying post-processing operation. 10 kg-scale manufacture has been successful with the method of the present disclosure.

Figure 1 shows a schematic diagram of an example of the method of the present disclosure using flow synthesis. The diagram shows that compound 1 ((R)-2-hydroxy-2-methyl-4-(2,4,5-trimethyl-3,6-dioxocyclohexa-1,4-dienyl)butanamide) is obtained by mixing compound 2 ((R)-6-hydroxy-2,5,7,8-tetramethylchroman-2-carboxamide) and an aqueous iron(III) chloride solution in a T-shaped mixer, allowing a reaction to progress in a tube reactor, and mixing the reactant with an aqueous sodium hydroxide solution in a subsequent T shaped mixture.

It is understood that the following equilibrium reaction occurs in the absence of a base.

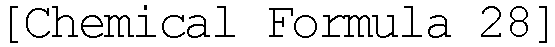

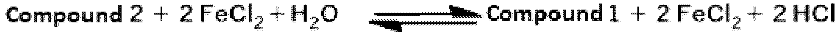

Since hydrochloric acid that is generated is captured in the presence of a base, the reaction to the right side is accelerated to result in the following reaction.

Compound 2 + 2FeCl₃ + H₂O + 2NaOH → compound 1 + 2FeCl₂ + 2NaCl + 2H₂O

The following abbreviations can also be used in the Examples and the Tables therein to simplify the descriptions of the specification.
PEA: 1-phenylethylamine
EtOAc: ethyl acetate
*ⁱ*PrOAc: isopropyl acetate
NMP: N-methylpyrrolidone
DMA: dimethylacetamide
DMF: N,N-dimethylformamide
MTBE: methyl tert-butyl ether
DME: 1,2-dimethoxyethane
2-MeTHF: 2-methyltetrahydrofuran
MEK: methyl ethyl ketone
DMSO: dimethyl sulfoxide
CDCl₃: deuterated chloroform
MeOH: methanol
MeCN: acetonitrile
IPA: 2-propanol
CPME: cyclopentyl methyl ether
Trolox amide: (R)-6-hydroxy-2,5,7,8-tetramethylchroman-2-carboxamide For symbols used in NMR, δ means chemical shift, s means singlet, d means doublet, t means triplet, q means quartet, m means multiplet, and J means spin coupling constant.

The following analysis conditions were used in HPLC (high performance liquid chromatography) analysis.

### (Reagent/sample solution)

Acetonitrile: for liquid chromatography (FUJIFILM Wako Pure Chemical) or equivalent
Trifluoroacetic acid: special grade (FUJIFILM Wako Pure Chemical) or equivalent
Water: water manufactured for testing with an ultrapure water manufacturing system, etc.

### (Solvent)

### Acetonitrile

### (Mobile phase)

### Mobile phase A: water/trifluoroacetic acid mixture (2000:1)

<Preparation Example> 2000 mL of water and 1 mL of trifluoroacetic acid are admixed and deaerated using an ultrasound washer.

### Mobile phase B: acetonitrile/trifluoroacetic acid mixture (2500:1)

<Preparation Example> 2500 mL of acetonitrile and 1 mL of trifluoroacetic acid are admixed and deaerated using an ultrasound washer.

### (Syringe detergent)

### Acetonitrile

### 1. Apparatus and setting/conditions

### 1.1 Apparatus

High performance chromatograph: UFLCXR (Shimadzu) or equivalent
Electronic balance: XP205DRV (Mettler Toledo) or equivalent
Ultrapure water manufacturing system: Milli-Q Advantage A10 (Merck) or equivalent
Ultrasound washer: US-4R (AS ONE) or equivalent

### 1.2 Setting/condition for liquid chromatography

Detector: ultraviolet absorption spectrophotometer (measurement wavelength: 235 nm)
Column: stainless tube with an inner diameter of 4.6 mm and a length of 150 mm is loaded with octadecylsilyl silica gel for liquid
chromatography with a particle size of 3.5 µm
[Zorbax SB C18 (Agilent) or equivalent]
Column temperature: constant temperature near 35°C
Mobile phase A: water/trifluoroacetic acid mixture (2000:1)
Mobile phase B: acetonitrile/trifluoroacetic acid mixture (2500:1)
Delivery of mobile phase: the mixing ratio of mobile phase A to mobile phase B is changed in the following manner to control the concentration gradient.

**[Table 1]**

| Time after infusion (min) | Mobile phase A (vol%) | Mobile phase B (vol%) |
|---|---|---|
| 0 to 25.0 | 98 → 0 | 2 → 100 |
| 25.0 to 33.0 | 0 | 100 |
| 33.0 to 33.1 | 0 → 98 | 100 → 2 |
| 33.1 to 40.0 | 98 | 2 |

| | | |
|---|---|---|
| Flow rate: 1.0 mL per minute | | |

Area measurement range: 33 minutes after infusion of sample solution (data collection time is 40 minutes)
Amount infused: 5 µL
Sample cooler temperature: constant temperature near 25°C Mixer volume: 0.5 mL
Syringe detergent: acetonitrile
Sample concentration: 2.5 µg/mL to 0.5 mg/mL

### <Example of waveform processing parameter setting>

Minimum area: 5000 µV*second
Minimum height: 100 µV
Detection sensitivity: 50 µV/second
Peak width: 1 second
Output intensity range: -500 to 1000 mAU
Output time range: 0 to 33 minutes

### [Examples]

### (Example 1)

### Elucidation of reaction mechanism of an oxidation reaction using iron(III) chloride (3)

To an isopropyl acetate solution (2 mL) of (R)-6-hydroxy-2,5,7,8-tetramethylchroman-2-carboxamide (200 mg, 0.802 mmol), 4.2 equivalents of iron(III) chloride hexahydrate (910 mg, 3.37 mol) were added at 30°C or lower, and 2.1 equivalents of sodium hydroxide (67.4 mg, 1.69 mmol) were further added. After stirring for 1 hour, the degree of loss of the raw materials was examined, and the remaining (R)-6-hydroxy-2,5,7,8-tetramethylchroman-2-carboxamide was 0.2%..

### (Example 2)

### Elucidation of reaction mechanism of an oxidation reaction using iron(III) chloride (4)

To an isopropyl acetate solution (2 mL) of (R)-6-hydroxy-2,5,7,8-tetramethylchroman-2-carboxamide (200 mg, 0.802 mmol), 4.2 equivalents of iron(III) chloride hexahydrate (910 mg, 3.37 mol) were added at 30°C or lower, and 2.1 equivalents of base (1.69 mmol) were further added. After stirring for 1 hour, the degree of loss of the raw materials was investigated, and the amount of residual (R)-6-hydroxy-2,5,7,8-tetramethylchroman-2-carboxamide was 0.2% or less.

**[Table 2]**

| Added base | Raw material ratio |
|---|---|
| Triethylamine | 0.1% |
| Potassium carbonate | 0.2% |
| Sodium carbonate | 0.1% |
| Sodium hydrogen carbonate | 0.1% |
| 28% Aqueous ammonia solution | Less than 0.1% |

### (Example 3)

### Investigation of solubility of (R)-6-hydroxy-2,5,7,8-tetramethylchroman-2-carboxamide

The solubility of (R)-6-hydroxy-2,5,7,8-tetramethylchroman-2-carboxamide was studied.

**[Table 3]**

| | | | | | Values in percentage: g/100 g | | |
|---|---|---|---|---|---|---|---|
| Temperature (°C) | 0 | 20 | 40 | 50 | 60 | 80 | 100 |
| Isopropyl acetate | 0.68 | 1.07 | 1.91 | | 3.01 | 4.71 | |
| Ethyl acetate | 0.83 | 1.28 | 2.24 | | 3.97 | | |
| THF | 7.08 | 13.6 | 18.9 | | 22.5 | | |
| 2-methyl-THF | 2.15 | 3.52 | 5.51 | | 6.37 | | |
| Acetone | 3.10 | 4.55 | 7.29 | 12.6 | | | |
| Toluene | 0.048 | 0.12 | 0.18 | | 0.45 | 1.22 | 2.17 |
| Anisole | 0.11 | 0.25 | 0.60 | | 1.42 | 3.93 | 5.78 |
| MTBE | 0.23 | 0.35 | 0.59 | 0.78 | | | |
| CPME | 0.35 | 0.61 | 0.95 | | 1.77 | 3.34 | 4.48 |
| Methanol | 4.07 | 6.11 | 9.69 | | 16.2 | | |
| Ethanol | 2.81 | 5.16 | 7.91 | | 15.3 | | |
| 2-propanol (IPA) | 1.56 | 3.48 | 4.14 | | 5.59 | 11.6 | |
| 4-methyltetrahydropyran | 0.65 | 2.12 | 3.16 | | 5.53 | 11.0 | 20.2 |
| Acetonitrile | 0.72 | 1.53 | 3.20 | | 6.47 | | |
| NMP | 6.03 | 11.2 | 10.6 | | 14.7 | | |
| DMSO | - | 11.0 | 11.5 | | 14.7 | | |

Among the solvents described above, THF exhibited the highest solubility at 20°C. The following oxidation reactions were conducted by using DMSO, which has higher safety, as a solvent from the viewpoint of safety as a solvent used in an oxidation reaction.

### (Example 4)

### Oxidation reaction using an aqueous iron(III) chloride solution of (R)-6-hydroxy-2,5,7,8-tetramethylchroman-2-carboxamide solution using DMSO as a solvent in a batch reactor

To a DMSO solution (10 mL) of (R)-6-hydroxy-2,5,7,8-tetramethylchroman-2-carboxamide (1 g, 4.01 mmol), 4.2 equivalents of iron(III) chloride hexahydrate (4.0 mol/L, 4.21 mL, 16.84 mmol) were added at 30°C or lower, and 4.0 equivalents of aqueous sodium hydroxide solution (5.0 mol/L, 3.21 mL, 16.05 mmol) were further added, and the mixture was vigorously stirred. After 1 hour of stirring, the degree of loss of the raw materials was examined, and (R)-6-hydroxy-2,5,7,8-tetramethylchroman-2-carboxamide decreased to 0.55%, and (R)-2-hydroxy-2-methyl-4-(2,4,5-trimethyl-3,6-dioxocyclohexa-1,4-dienyl)butanamide was produced as the major product.

However, this approach involves a risk of complete solidification in a flask if the mixture cannot be vigorously stirred. As a result of difficulty in stirring, there is also a high risk of difficulty in subsequent retrieval. In view of the above, it is desirable to employ an approach that can complete the reaction by using a flow reactor.

### (Example 5)

### Oxidation reaction using an aqueous iron(III) chloride solution of (R)-6-hydroxy-2,5,7,8-tetramethylchroman-2-carboxamide using DMSO as a solvent in a flow reactor

A DMSO solution of 0.5 mol/L (R)-6-hydroxy-2,5,7,8-tetramethylchroman-2-carboxamide was prepared (reaction solution A). An aqueous 4.0 mol/L iron(III) chloride solution was prepared (reaction solution B). An aqueous 5.0 mol/L sodium hydroxide solution was prepared (reaction solution C). Reaction solution A was delivered at a flow rate of 10 mL/min and reaction solution B was delivered at a flow rate of 5.25 mL/min by using a tube with an inner diameter of 1.0 mm, and the solutions were mixed in a T-shaped mixer (mixer 1) with an inner diameter of 1.0 mm. After passing mixer 1, the mixture was mixed with reaction solution C delivered by the same approach in a second T-shape mixture (inner diameter 1.0 mm, mixer 2) by using a tube with an inner diameter of 1.0 mm. The residence time between mixer 1 and mixer 2 was set to be any time between 0.1 seconds and 10 seconds. The reaction solution discharged through mixer 2 was discharged from a flow reactor system using a tube with an inner diameter of 1.0 mm to perform sampling. The solution was delivered while immersing and heating mixer 1, mixer 2, and connecting tube in a 75°C heating bath. It was confirmed that the residual ratio of (R)-6-hydroxy-2,5,7,8-tetramethylchroman-2-carboxamide was 2% or less, and the desired product of (R)-2-hydroxy-2-methyl-4-(2,4,5-trimethyl-3,6-dioxocyclohexa-1,4-dienyl)butanamide was produced at a production rate of 91%.

### (Example 6)

### Oxidation reaction using an aqueous iron(III) chloride solution of (R)-6-hydroxy-2,5,7,8-tetramethylchroman-2-carboxamide using DMSO as a solvent in a flow reactor and isolation of a product

A DMSO solution of 0.5 mol/L (R)-6-hydroxy-2,5,7,8-tetramethylchroman-2-carboxamide was prepared (reaction solution A). An aqueous 4.0 mol/L iron(III) chloride solution was prepared (reaction solution B). An aqueous 5.0 mol/L sodium hydroxide solution was prepared (reaction solution C). Reaction solution A was delivered at a flow rate of 10 mL/min and reaction solution B was delivered at a flow rate of 5.25 mL/min by using a tube with an inner diameter of 1.0 mm, and the solutions were mixed in a T-shaped mixer (mixer 1) with an inner diameter of 1.0 mm. After passing mixer 1, the mixture was mixed with reaction solution C delivered by the same approach in a second T-shape mixture (inner diameter 1.0 mm, mixer 2) by using a tube with an inner diameter of 1.0 mm. The residence time between mixer 1 and mixer 2 was set to be any time between 0.1 seconds and 10 seconds. The reaction solution discharged through mixer 2 was discharged from a flow reactor system using a tube with an inner diameter of 1.0 mm to perform sampling. The solution was delivered while immersing and heating mixer 1, mixer 2, and connecting tube in a 75°C heating bath. It was confirmed that the residual ratio of (R)-6-hydroxy-2,5,7,8-tetramethylchroman-2-carboxamide was 2% or less, and the desired product of (R)-2-hydroxy-2-methyl-4-(2,4,5-trimethyl-3,6-dioxocyclohexa-1,4-dienyl)butanamide was produced at a production rate of 91%. The reaction solution discharged from a flow reactor was retrieved for 18 minutes and 3 seconds in a receiving container regulated to have a temperature of 40°C, and 90 g of 1 mol/L hydrochloric acid water was added dropwise. The mixture was then cooled to 20°C, and 18 g of concentrated hydrochloric acid was added dropwise. The pH was confirmed to be 0.5 or less. 90 g of water was then further added dropwise, and the resulting crystal was filtered. The wet crystal was washed twice with 67.5 g of 1 mol/L of hydrochloric acid water, and washed three times with 45 g of water. The crystal was dried with a vacuum dryer heated to 60°C to obtain 20.35 g of (R)-2-hydroxy-2-methyl-4-(2,4,5-trimethyl-3,6-dioxocyclohexa-1,4-dienyl)butanamide.

### (Comparative Example 1)

### Elucidation of reaction mechanism of an oxidation reaction using iron(III) chloride (1)

To an isopropyl acetate solution (2 mL) of (R)-6-hydroxy-2,5,7,8-tetramethylchroman-2-carboxamide (200 mg, 0.802 mmol), 2.1 equivalents of iron(III) chloride hexahydrate (455 mg, 1.69 mol) were added at 30°C or lower. When the degree of loss of raw materials after stirring for 1 hour was investigated, 73% of (R)-6-hydroxy-2,5,7,8-tetramethylchroman-2-carboxamide was remaining.

### (Comparative Example 2)

### Elucidation of reaction mechanism of an oxidation reaction using iron(III) chloride (2)

To an isopropyl acetate solution (2 mL) of (R)-6-hydroxy-2,5,7,8-tetramethylchroman-2-carboxamide (200 mg, 0.802 mmol), 4.2 equivalents of iron(III) chloride hexahydrate (910 mg, 3.37 mol) were added at 30°C or lower. When the degree of loss of raw materials after stirring for 1 hour was investigated, 21% of (R)-6-hydroxy-2,5,7,8-tetramethylchroman-2-carboxamide was remaining.

As described above, the present disclosure is exemplified by the use of its preferred embodiments. It is understood that the scope of the present disclosure should be interpreted solely based on the Claims. It is understood that any patent, any patent application, and any references cited herein should be incorporated herein by reference in the same manner as the contents are specifically described herein. This application is based on a patent application No. 2022-158569 filed in Japan (filing date: September 30, 2022), the contents of which are incorporated in full herein.

### Industrial Applicability

The present disclosure is useful in the manufacture of medicaments.

## Claims

1. A method of manufacturing a compound of formula I: 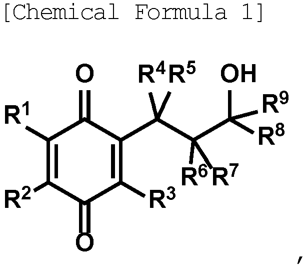 comprising the steps of:
contacting, in a solvent, an oxidizing agent with a compound of formula II: 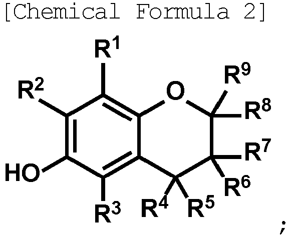 and
contacting a base with the solvent to promote a reaction between the compound of formula II and the oxidizing agent;
wherein
R¹, R², R³, R⁴, R⁵, R⁶, R⁷, R⁸, and R⁹ each independently represent a hydrogen atom, halogen, optionally substituted C₁₋₆ alkyl, optionally substituted C₁₋₆ alkoxy, or -C(=O)NR^{A}R^{B}, and
R^{A} and R^{B} each independently represent a hydrogen atom, optionally substituted C₁₋₆ alkyl, or optionally substituted C₁₋₆ alkoxy.

2. The method of claim 1, wherein R¹ is a hydrogen atom or optionally substituted C₁₋₆ alkyl.

3. The method of claim 1 or 2, wherein R¹ is methyl.

4. The method of any one of claims 1 to 3, wherein R² is a hydrogen atom or optionally substituted C₁₋₆ alkyl.

5. The method of any one of claims 1 to 4, wherein R² is methyl.

6. The method of any one of claims 1 to 5, wherein R³ is a hydrogen atom or optionally substituted C₁₋₆ alkyl.

7. The method of any one of claims 1 to 6, wherein R³ is methyl.

8. The method of any one of claims 1 to 7, wherein R⁴ is a hydrogen atom or optionally substituted C₁₋₆ alkyl.

9. The method of any one of claims 1 to 8, wherein R⁴ is a hydrogen atom.

10. The method of any one of claims 1 to 9, wherein R⁵ is a hydrogen atom or optionally substituted C₁₋₆ alkyl.

11. The method of any one of claims 1 to 10, wherein R⁵ is a hydrogen atom.

12. The method of any one of claims 1 to 11, wherein R⁶ is a hydrogen atom or optionally substituted C₁₋₆ alkyl.

13. The method of any one of claims 1 to 12, wherein R⁶ is a hydrogen atom.

14. The method of any one of claims 1 to 13, wherein R⁷ is a hydrogen atom or optionally substituted C₁₋₆ alkyl.

15. The method of any one of claims 1 to 14, wherein R⁷ is a hydrogen atom.

16. The method of any one of claims 1 to 15, wherein R⁸ is a hydrogen atom, optionally substituted C₁₋₆ alkyl, or -C(=O)NR^{A}R^{B}.

17. The method of any one of claims 1 to 16, wherein R⁸ is - C(=O)NR^{A}R^{B}.

18. The method of any one of claims 1 to 17, wherein R⁹ is a hydrogen atom or optionally substituted C₁₋₆ alkyl.

19. The method of any one of claims 1 to 18, wherein R⁹ is methyl.

20. The method of any one of claims 1 to 19, wherein R^{A} and R^{B} each represent a hydrogen atom.

21. The method of any one of claims 1 to 20, wherein the solvent is a polar aprotic solvent.

22. The method of any one of claims 1 to 20, wherein the solvent is a polar protic solvent.

23. The method of any one of claims 1 to 20, wherein the solvent is selected from the group consisting of isopropyl acetate, ethyl acetate, tetrahydrofuran, 2-methyl-tetrahydrofuran, acetone, toluene, anisole, methyl tert-butyl ether, cyclopentyl methyl ether, methanol, ethanol, 2-propanol, 4-methyltetrahydropyran, acetonitrile, N-methylpyrrolidone, and dimethylsulfoxide.

24. The method of any one of claims 1 to 23, wherein the solvent is dimethylsulfoxide.

25. The method of any one of claims 1 to 24, wherein the oxidizing agent comprises a compound comprising iron.

26. The method of any one of claims 1 to 25, wherein the oxidizing agent is iron(III) chloride or iron(III) nitrate.

27. The method of any one of claims 1 to 26, wherein the oxidizing agent is iron(III) chloride hexahydrate.

28. The method of any one of claims 1 to 27, wherein the base is selected from the group consisting of sodium hydroxide, potassium hydroxide, calcium hydroxide, barium hydroxide, potassium carbonate, sodium carbonate, sodium hydrogen carbonate, triethylamine, diisopropylethylamine, and ammonia water.

29. The method of any one of claims 1 to 28, wherein the base is selected from the group consisting of sodium hydroxide, potassium carbonate, sodium carbonate, sodium hydrogen carbonate, triethylamine, and ammonia water.

30. The method of claim 29, wherein the base is sodium hydroxide.

31. The method of any one of claims 1 to 30, wherein the step of contacting the oxidizing agent is performed in a flow reactor.

32. The method of claim 31, wherein an inner diameter of the flow reactor is about 0.1 mm to about 100 mm in the step of contacting the oxidizing agent.

33. The method of claim 31 or 32, wherein a residence time of the step of contacting the oxidizing agent is 60 seconds or less.

34. The method of claim 33, wherein the residence time is 0.1 seconds to 10 seconds.

35. The method of any one of claims 1 to 34, wherein the step of contacting the oxidizing agent is performed at a temperature from 0°C to 100°C.

36. The method of claim 35, wherein the step of contacting the oxidizing agent is performed at a temperature of 75°C or higher.

37. The method of claim 35, wherein the step of contacting the oxidizing agent is performed at a temperature from 60°C to 90°C.

38. The method of any one of claims 1 to 37, wherein the step of contacting the base is performed after the step of contacting the oxidizing agent.

39. The method of any one of claims 1 to 38, wherein the step of contacting the base is performed by contacting a base with a reaction mixture produced by the step of contacting the oxidizing agent.

40. The method of any one of claims 1 to 39, wherein the oxidizing agent is in the solvent in the step of contacting the base.

41. The method of any one of claims 1 to 40, wherein the step of contacting the base is performed in a flow reactor.

42. The method of claim 41, wherein an inner diameter of the flow reactor is about 0.1 mm to about 100 mm in the step of contacting the base.

43. The method of claim 41 or 42, wherein a residence time of the step of contacting the base is 60 seconds or less.

44. The method of claim 43, wherein the residence time is 0.1 seconds to 10 seconds.

45. The method of any one of claims 1 to 44, wherein the step of contacting the base is performed at a temperature from 0°C to 100°C.

46. The method of claim 45, wherein the step of contacting the base is performed at a temperature from 60°C to 90°C.

47. The method of claim 45, wherein the step of contacting the oxidizing agent is performed at a temperature of 75°C or higher.

48. The method of any one of claims 1 to 47, wherein a carbon atom, attached with R⁸, R⁹, and OH, is asymmetric carbon in the compound of formula I.

49. The method of claim 48, wherein the compound of formula II is an optically active substance and optical purity is maintained in a reaction between the compound of formula II and the oxidizing agent.

50. The method of any one of claims 1 to 49, wherein the compound of formula I is 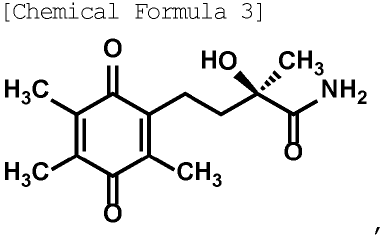 and
the compound of formula II is

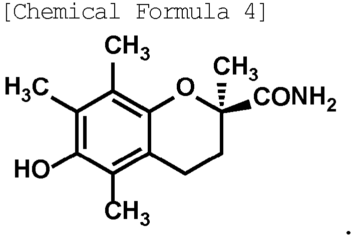

51. The method of claim 50, wherein the compound of formula II is manufactured by the step of optically resolving and amidating a compound of formula III:
